# EUROPEAN PATENT APPLICATION

(11) **EP 3 828 200 A1**
(43) Date of publication of application: **02.06.2021**
(21) Application number: 19833025.0
(22) Date of filing: 08.07.2019
(51) Int. Cl.: C07K 16/00, C12N 5/10, C12N 15/13, C12N 15/19, C12N 15/21, C12P 21/02

(54) **CYCLIC SINGLE-CHAIN ANTIBODY**

(30) Priority: 09.07.2018 JP 2018130203
(71) Applicant: National University Corporation Kumamoto University, Kumamoto-shi, Kumamoto 860-8555 (JP)
(72) Inventor: MORIOKA, Hiroshi, Kumamoto-shi, Kumamoto 862-0973 (JP); KOBASHIGAWA, Yoshihiro, Kumamoto-shi, Kumamoto 862-0973 (JP); SATO, Takashi, Kumamoto-shi, Kumamoto 862-0973 (JP); FUKUDA, Natsuki, Kumamoto-shi, Kumamoto 862-0973 (JP); YAMAUCHI, Soichiro, Kumamoto-shi, Kumamoto 862-0973 (JP)
(74) Representative: Plasseraud IP
(86) International application number: PCT/JP2019/026983
(87) International publication number: WO 2020/013126

(57) **Abstract**

The present invention provides an scFv comprising a heavy chain variable region (VH) and a light chain variable region linked by a first peptide linker, wherein an N-terminus and a C-terminus thereof are linked by a second peptide linker.

SEQUENCE LISTING

<110>  NATIONAL UNIVERSITY CORPORATION KUMAMOTO UNIVERSITY

<120>  Cyclic single chain antibody

<130>  15078AA10/FA5009-19167

<150>  JP 2018-130203
<151>  2018-07-09

<160>  16

<170>  PatentIn version 3.5

<210>  1
<211>  289
<212>  PRT
<213>  Artificial Sequence

<220>
<223>  cyclic single chain antibody cyclized by amide bond formation
       between N and C terminals

<400>  1

Met Ser Ser His His His His His His Leu Glu Val Leu Phe Gln Gly
1               5                   10                  15

Gly Gly Ser His Met Ala Gln Val Lys Leu Gln Gln Ser Gly Pro Ser
            20                  25                  30

Leu Val Lys Pro Ser Gln Thr Leu Ser Leu Thr Cys Ser Val Thr Gly
        35                  40                  45

Asp Ser Ile Thr Ser Gly Tyr Trp Asn Trp Ile Arg Lys Phe Pro Gly
    50                  55                  60

Asn Lys Phe Glu Tyr Leu Gly Tyr Ile Ser Tyr Ser Gly Arg Thr Tyr
65                  70                  75                  80

Tyr Asn Pro Ser Leu Lys Ser Arg Ile Ser Ile Thr Arg Asp Thr Ser
                85                  90                  95

Lys Asn Gln Tyr Tyr Leu Gln Leu Asn Ser Val Thr Thr Glu Asp Thr
            100                 105                 110

Ala Thr Tyr Tyr Cys Ser Arg Pro Tyr Tyr Arg Tyr Asp Tyr Ala Ile
        115                 120                 125

Asp Tyr Trp Gly Gln Gly Thr Thr Val Thr Val Ser Ser Gly Gly Gly
    130                 135                 140

Gly Ser Gly Gly Gly Gly Ser Gly Gly Gly Gly Ser Asp Ile Glu Leu

145                 150                 155                 160

Thr Gln Ser Pro Ala Ile Met Ser Ala Ser Leu Gly Glu Gln Val Thr
                165                 170                 175

Met Thr Cys Thr Ala Ser Ser Ser Val Ser Ser Ser Tyr Leu His Trp
            180                 185                 190

Tyr Gln Gln Lys Pro Gly Ser Ser Pro Lys Leu Trp Ile Tyr Ser Thr
        195                 200                 205

Ser Asn Leu Ala Ser Gly Val Pro Ala Arg Phe Ser Ser Ser Gly Ser
    210                 215                 220

Gly Thr Ser Tyr Ser Leu Thr Ile Ser Arg Met Glu Ala Glu Asp Ala
225                 230                 235                 240

Ala Thr Tyr Tyr Cys Gln Gln Ser Trp Lys Ala Pro Tyr Thr Phe Gly
                245                 250                 255

Gly Gly Thr Lys Leu Glu Ile Lys Arg Ala Ala Ala Gly Thr His His
            260                 265                 270

His His His His Leu Pro Glu Thr Gly Leu Glu His His His His His
        275                 280                 285

His

<210>  2
<211>  870
<212>  DNA
<213>  Artificial Sequence

<220>
<223>  plasmid for Y9-scFv^LPETG

<400>  2
atgagcagcc atcatcatca tcatcatctg gaagtgctgt ttcagggcgg tggaagccat        60

atggcccagg tgaaactgca gcagtcagga cctagcctcg tgaaaccttc tcagactctg       120

tccctcacct gttctgtcac tggcgactcc atcaccagtg gttactggaa ctggatccgg       180

aaattcccag ggaataaatt tgagtacttg ggttacataa gctacagtgg tcgcacttac       240

tacaatccat ctctcaaaag tcgaatctcc atcactcgag acacatccaa gaaccagtac       300

tacctgcagt tgaattctgt gactactgag gacacagcca catattactg ttcaagaccc       360

tactataggt acgactatgc tatagactac tggggccaag ggaccacggt caccgtctcc       420

tcaggtggag gcggttcagg cggaggtggc tctggcggtg gcggatcgga catcgagctc       480

actcagtctc cagcaatcat gtctgcatct ctaggggaac aggtcaccat gacctgcact 540

gccagctcaa gtgtaagttc cagttacttg cactggtacc agcagaagcc aggatcctcc 600

cccaaactct ggatttatag cacatccaac ctggcttctg gagtcccagc tcgcttcagt 660

agcagtggat ctgggacctc ttactctctc acaatcagcc gaatggaggc tgaagatgct 720

gccacctatt actgccagca aagttggaag gccccgtaca cgttcggagg ggggacaaag 780

ttggaaataa aacgggcggc cgcaggtacc catcatcatc atcatcatct gccggaaacc 840

ggcctcgagc accaccacca ccaccactga 870

<210> 3
<211> 255
<212> PRT
<213> Artificial Sequence

<220>
<223> non-cyclic Tras-scFv

<400> 3

Met Glu Val Gln Leu Val Glu Ser Gly Gly Gly Leu Val Gln Pro Gly
1               5                   10                  15

Gly Ser Leu Arg Leu Ser Cys Ala Ala Ser Gly Phe Asn Ile Lys Asp
            20                  25                  30

Thr Tyr Ile His Trp Val Arg Gln Ala Pro Gly Lys Gly Leu Glu Trp
        35                  40                  45

Val Ala Arg Ile Tyr Pro Thr Asn Gly Tyr Thr Arg Tyr Ala Asp Ser
    50                  55                  60

Val Lys Gly Arg Phe Thr Ile Ser Ala Asp Thr Ser Lys Asn Thr Ala
65                  70                  75                  80

Tyr Leu Gln Met Asn Ser Leu Arg Ala Glu Asp Thr Ala Val Tyr Tyr
                85                  90                  95

Cys Ser Arg Trp Gly Gly Asp Gly Phe Tyr Ala Met Asp Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Asp Ile Gln Met Thr Gln Ser Pro
    130                 135                 140

Ser Ser Leu Ser Ala Ser Val Gly Asp Arg Val Thr Ile Thr Cys Arg

145                 150                 155                 160

Ala Ser Gln Asp Val Asn Thr Ala Val Ala Trp Tyr Gln Gln Lys Pro
                165                 170                 175

Gly Lys Ala Pro Lys Leu Leu Ile Tyr Ser Ala Ser Phe Leu Tyr Ser
            180                 185                 190

Gly Val Pro Ser Arg Phe Ser Gly Ser Arg Ser Gly Thr Asp Phe Thr
        195                 200                 205

Leu Thr Ile Ser Ser Leu Gln Pro Glu Asp Phe Ala Thr Tyr Tyr Cys
    210                 215                 220

Gln Gln His Tyr Thr Thr Pro Pro Thr Phe Gly Gln Gly Thr Lys Val
225                 230                 235                 240

Glu Ile Lys Ser Ala Ala Ala Leu Glu His His His His His His
                245                 250                 255

<210>  4
<211>  768
<212>  DNA
<213>  Artificial Sequence

<220>
<223>  coding non-cyclic Tras-scFv

<400>  4
atggaagtgc agctggttga aagcggtggc ggtctggtgc aaccgggcgg tagcctgcgt      60

ctgagctgcg cggcgagcgg ttttaacatc aaagacacct acattcactg ggttcgtcaa     120

gcgccgggca agggtctgga gtgggttgcg cgtatctatc cgaccaacgg ctacacccgt     180

tatgcggaca gcgtgaaagg tcgttttacc attagcgcgg ataccagcaa gaacaccgcg     240

tacctgcaga tgaacagcct gcgtgcggaa gacaccgcgg tttactattg cagccgttgg     300

ggcggtgacg gcttctacgc gatggattat tggggccaag gcaccctggt gaccgttagc     360

agcggcggtg gaggtagtgg tggtggaggt tccggtggtg gcggtagcga tatccagatg     420

acccagagcc cgagcagcct gagcgcgagc gtgggtgacc gtgttaccat tacctgccgt     480

gcgagccaag atgtgaacac cgcggttgcg tggtatcagc aaaagccggg caaagcgccg     540

aagctgctga tctacagcgc gagcttcctg tatagcggtg tgccgagccg ttttagcggc     600

agccgtagcg gcaccgactt caccctgacc attagcagcc tgcagccgga ggattttgcg     660

acctactatt gccagcaaca ctataccacc ccgccgacct ttggtcaagg caccaaagtt     720

gaaatcaaga gcgcggccgc actcgagcac caccaccacc accactga                  768

<210>  5
<211>  288
<212>  PRT
<213>  Artificial Sequence

<220>
<223>  Substrate of cyclization

<400>  5

Met Ser Ser His His His His His His Leu Glu Val Leu Phe Gln Gly
1               5                   10                  15

Gly Gly Ser His Met Glu Val Gln Leu Val Glu Ser Gly Gly Gly Leu
            20                  25                  30

Val Gln Pro Gly Gly Ser Leu Arg Leu Ser Cys Ala Ala Ser Gly Phe
        35                  40                  45

Asn Ile Lys Asp Thr Tyr Ile His Trp Val Arg Gln Ala Pro Gly Lys
    50                  55                  60

Gly Leu Glu Trp Val Ala Arg Ile Tyr Pro Thr Asn Gly Tyr Thr Arg
65                  70                  75                  80

Tyr Ala Asp Ser Val Lys Gly Arg Phe Thr Ile Ser Ala Asp Thr Ser
                85                  90                  95

Lys Asn Thr Ala Tyr Leu Gln Met Asn Ser Leu Arg Ala Glu Asp Thr
            100                 105                 110

Ala Val Tyr Tyr Cys Ser Arg Trp Gly Gly Asp Gly Phe Tyr Ala Met
        115                 120                 125

Asp Tyr Trp Gly Gln Gly Thr Leu Val Thr Val Ser Ser Gly Gly Gly
    130                 135                 140

Gly Ser Gly Gly Gly Gly Ser Gly Gly Gly Gly Ser Asp Ile Gln Met
145                 150                 155                 160

Thr Gln Ser Pro Ser Ser Leu Ser Ala Ser Val Gly Asp Arg Val Thr
                165                 170                 175

Ile Thr Cys Arg Ala Ser Gln Asp Val Asn Thr Ala Val Ala Trp Tyr
            180                 185                 190

Gln Gln Lys Pro Gly Lys Ala Pro Lys Leu Leu Ile Tyr Ser Ala Ser
        195                 200                 205

Phe Leu Tyr Ser Gly Val Pro Ser Arg Phe Ser Gly Ser Arg Ser Gly

210                 215                 220

Thr Asp Phe Thr Leu Thr Ile Ser Ser Leu Gln Pro Glu Asp Phe Ala
225                 230                 235                 240

Thr Tyr Tyr Cys Gln Gln His Tyr Thr Thr Pro Pro Thr Phe Gly Gln
                245                 250                 255

Gly Thr Lys Val Glu Ile Lys Ser Ala Ala Ala Gly Thr His His His
            260                 265                 270

His His His Leu Pro Glu Thr Gly Leu Glu His His His His His His
        275                 280                 285

<210>  6
<211>  264
<212>  PRT
<213>  Artificial Sequence

<220>
<223>  Cyclic Tras-scFv cyclized by amide bond formation between N and C
       terminals

<400>  6

Gly Gly Gly Ser His Met Glu Val Gln Leu Val Glu Ser Gly Gly Gly
1               5                   10                  15

Leu Val Gln Pro Gly Gly Ser Leu Arg Leu Ser Cys Ala Ala Ser Gly
            20                  25                  30

Phe Asn Ile Lys Asp Thr Tyr Ile His Trp Val Arg Gln Ala Pro Gly
        35                  40                  45

Lys Gly Leu Glu Trp Val Ala Arg Ile Tyr Pro Thr Asn Gly Tyr Thr
    50                  55                  60

Arg Tyr Ala Asp Ser Val Lys Gly Arg Phe Thr Ile Ser Ala Asp Thr
65                  70                  75                  80

Ser Lys Asn Thr Ala Tyr Leu Gln Met Asn Ser Leu Arg Ala Glu Asp
                85                  90                  95

Thr Ala Val Tyr Tyr Cys Ser Arg Trp Gly Gly Asp Gly Phe Tyr Ala
            100                 105                 110

Met Asp Tyr Trp Gly Gln Gly Thr Leu Val Thr Val Ser Ser Gly Gly
        115                 120                 125

Gly Gly Ser Gly Gly Gly Gly Ser Gly Gly Gly Gly Ser Asp Ile Gln

130                 135                 140

Met Thr Gln Ser Pro Ser Ser Leu Ser Ala Ser Val Gly Asp Arg Val
145                 150                 155                 160

Thr Ile Thr Cys Arg Ala Ser Gln Asp Val Asn Thr Ala Val Ala Trp
                165                 170                 175

Tyr Gln Gln Lys Pro Gly Lys Ala Pro Lys Leu Leu Ile Tyr Ser Ala
            180                 185                 190

Ser Phe Leu Tyr Ser Gly Val Pro Ser Arg Phe Ser Gly Ser Arg Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln His Tyr Thr Thr Pro Pro Thr Phe Gly
225                 230                 235                 240

Gln Gly Thr Lys Val Glu Ile Lys Ser Ala Ala Ala Gly Thr His His
                245                 250                 255

His His His His Leu Pro Glu Thr
            260

<210>  7
<211>  867
<212>  DNA
<213>  Artificial Sequence

<220>
<223>  Substrate of cyclization

<400>  7
atgagcagcc atcatcatca tcatcatctg gaagtgctgt ttcagggcgg tggaagccat      60

atggaagtgc agctggttga aagcggtggc ggtctggtgc aaccgggcgg tagcctgcgt     120

ctgagctgcg cggcgagcgg ttttaacatc aaagacacct acattcactg ggttcgtcaa     180

gcgccgggca agggtctgga gtgggttgcg cgtatctatc cgaccaacgg ctacacccgt     240

tatgcggaca gcgtgaaagg tcgttttacc attagcgcgg ataccagcaa gaacaccgcg     300

tacctgcaga tgaacagcct gcgtgcggaa gacaccgcgg tttactattg cagccgttgg     360

ggcggtgacg gcttctacgc gatggattat tggggccaag gcaccctggt gaccgttagc     420

agcggcggtg gcggtagcgg cggtggcggt agcggcggtg gcggtagcga tatccagatg     480

acccagagcc cgagcagcct gagcgcgagc gtgggtgacc gtgttaccat tacctgccgt     540

gcgagccaag atgtgaacac cgcggttgcg tggtatcagc aaaagccggg caaagcgccg     600

aagctgctga tctacagcgc gagcttcctg tatagcggtg tgccgagccg ttttagcggc 660

agccgtagcg gcaccgactt caccctgacc attagcagcc tgcagccgga ggattttgcg 720

acctactatt gccagcaaca ctataccacc ccgccgacct ttggtcaagg caccaaagtt 780

gaaatcaaga gcgcggccgc aggtacccat catcatcatc atcatctgcc ggaaaccggc 840

ctcgagcacc accaccacca ccactga 867

<210> 8
<211> 325
<212> PRT
<213> Artificial Sequence

<220>
<223> GST-Her2

<400> 8

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu

165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Val Asn Cys Ser Gln Phe Leu Arg Gly Gln Glu Cys Val Glu
225                 230                 235                 240

Glu Cys Arg Val Leu Gln Gly Leu Pro Arg Glu Tyr Val Asn Ala Arg
                245                 250                 255

His Cys Leu Pro Cys His Pro Glu Cys Gln Pro Gln Asn Gly Ser Val
            260                 265                 270

Thr Cys Phe Gly Pro Glu Ala Asp Gln Cys Val Ala Cys Ala His Tyr
        275                 280                 285

Lys Asp Pro Pro Phe Cys Val Ala Arg Cys Pro Ser Gly Val Lys Pro
    290                 295                 300

Asp Leu Ser Tyr Met Pro Ile Trp Lys Phe Pro Asp Glu Glu Gly Ala
305                 310                 315                 320

Cys Gln Pro Leu Glu
                325

<210>  9
<211>  978
<212>  DNA
<213>  Artificial Sequence

<220>
<223>  coding GST-Her2

<400>  9
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa 420

acatatttaa atggtgatca tgtaacccat cctgacttca tgttgtatga cgctcttgat 480

gttgttttat acatggaccc aatgtgcctg gatgcgttcc caaaattagt ttgttttaaa 540

aaacgtattg aagctatccc acaaattgat aagtacttga aatccagcaa gtatatagca 600

tggcctttgc agggctggca agccacgttt ggtggtggcg accatcctcc aaaatcggat 660

ctggttccgc gtggatccgt caactgcagc cagttccttc ggggccagga gtgcgtggag 720

gaatgccgag tactgcaggg gctccccagg gagtatgtga atgccaggca ctgtttgccg 780

tgccaccctg agtgtcagcc ccagaatggc tcagtgacct gttttggacc ggaggctgac 840

cagtgtgtgg cctgtgccca ctataaggac cctcccttct gcgtggcccg ctgccccagc 900

ggtgtgaaac ctgacctctc ctacatgccc atctggaagt ttccagatga ggagggcgca 960

tgccagcctc tcgagtga 978

<210> 10
<211> 781
<212> PRT
<213> Artificial Sequence

<220>
<223> Substrate of cyclization

<400> 10

Met Ala Lys Thr Glu Glu Gly Lys Leu Val Ile Trp Ile Asn Gly Asp
1               5                   10                  15

Lys Gly Tyr Asn Gly Leu Ala Glu Val Gly Lys Lys Phe Glu Lys Asp
            20                  25                  30

Thr Gly Ile Lys Val Thr Val Glu His Pro Asp Lys Leu Glu Glu Lys
        35                  40                  45

Phe Pro Gln Val Ala Ala Thr Gly Asp Gly Pro Asp Ile Ile Phe Trp
    50                  55                  60

Ala His Asp Arg Phe Gly Gly Tyr Ala Gln Ser Gly Leu Leu Ala Glu
65                  70                  75                  80

Ile Thr Pro Asp Lys Ala Phe Gln Asp Lys Leu Tyr Pro Phe Thr Trp
                85                  90                  95

Asp Ala Val Arg Tyr Asn Gly Lys Leu Ile Ala Tyr Pro Ile Ala Val
            100                 105                 110

Glu Ala Leu Ser Leu Ile Tyr Asn Lys Asp Leu Leu Pro Asn Pro Pro

115                 120                 125

Lys Thr Trp Glu Glu Ile Pro Ala Leu Asp Lys Glu Leu Lys Ala Lys
    130                 135                 140

Gly Lys Ser Ala Leu Met Phe Asn Leu Gln Glu Pro Tyr Phe Thr Trp
145                 150                 155                 160

Pro Leu Ile Ala Ala Asp Gly Gly Tyr Ala Phe Lys Tyr Glu Asn Gly
                165                 170                 175

Lys Tyr Asp Ile Lys Asp Val Gly Val Asp Asn Ala Gly Ala Lys Ala
            180                 185                 190

Gly Leu Thr Phe Leu Val Asp Leu Ile Lys Asn Lys His Met Asn Ala
        195                 200                 205

Asp Thr Asp Tyr Ser Ile Ala Glu Ala Ala Phe Asn Lys Gly Glu Thr
    210                 215                 220

Ala Met Thr Ile Asn Gly Pro Trp Ala Trp Ser Asn Ile Asp Thr Ser
225                 230                 235                 240

Lys Val Asn Tyr Gly Val Thr Val Leu Pro Thr Phe Lys Gly Gln Pro
                245                 250                 255

Ser Lys Pro Phe Val Gly Val Leu Ser Ala Gly Ile Asn Ala Ala Ser
            260                 265                 270

Pro Asn Lys Glu Leu Ala Lys Glu Phe Leu Glu Asn Tyr Leu Leu Thr
        275                 280                 285

Asp Glu Gly Leu Glu Ala Val Asn Lys Asp Lys Pro Leu Gly Ala Val
    290                 295                 300

Ala Leu Lys Ser Tyr Glu Glu Glu Leu Ala Lys Asp Pro Arg Ile Ala
305                 310                 315                 320

Ala Thr Met Glu Asn Ala Gln Lys Gly Glu Ile Met Pro Asn Ile Pro
                325                 330                 335

Gln Met Ser Ala Phe Trp Tyr Ala Val Arg Thr Ala Val Ile Asn Ala
            340                 345                 350

Ala Ser Gly Arg Gln Thr Val Asp Glu Ala Leu Lys Asp Ala Gln Thr
        355                 360                 365

Asn Ser Ser Ser Leu Glu Val Leu Phe Gln Gly Pro His Met Ile Lys
    370                 375                 380

Ile Ala Thr Arg Lys Tyr Leu Gly Lys Gln Asn Val Tyr Asp Ile Gly
385                 390                 395                 400

Val Glu Arg Asp His Asn Phe Ala Leu Lys Asn Gly Phe Ile Ala Ser
                405                 410                 415

Asn Cys Phe Asn Gly Thr His His His His His His Ala Ala Ala Glu
            420                 425                 430

Val Gln Leu Val Glu Ser Gly Gly Gly Leu Val Gln Pro Gly Gly Ser
        435                 440                 445

Leu Arg Leu Ser Cys Ala Ala Ser Gly Phe Asn Ile Lys Asp Thr Tyr
    450                 455                 460

Ile His Trp Val Arg Gln Ala Pro Gly Lys Gly Leu Glu Trp Val Ala
465                 470                 475                 480

Arg Ile Tyr Pro Thr Asn Gly Tyr Thr Arg Tyr Ala Asp Ser Val Lys
                485                 490                 495

Gly Arg Phe Thr Ile Ser Ala Asp Thr Ser Lys Asn Thr Ala Tyr Leu
            500                 505                 510

Gln Met Asn Ser Leu Arg Ala Glu Asp Thr Ala Val Tyr Tyr Cys Ser
        515                 520                 525

Arg Trp Gly Gly Asp Gly Phe Tyr Ala Met Asp Tyr Trp Gly Gln Gly
    530                 535                 540

Thr Leu Val Thr Val Ser Ser Gly Gly Gly Gly Ser Gly Gly Gly Gly
545                 550                 555                 560

Ser Gly Gly Gly Gly Ser Asp Ile Gln Met Thr Gln Ser Pro Ser Ser
                565                 570                 575

Leu Ser Ala Ser Val Gly Asp Arg Val Thr Ile Thr Cys Arg Ala Ser
            580                 585                 590

Gln Asp Val Asn Thr Ala Val Ala Trp Tyr Gln Gln Lys Pro Gly Lys
        595                 600                 605

Ala Pro Lys Leu Leu Ile Tyr Ser Ala Ser Phe Leu Tyr Ser Gly Val
    610                 615                 620

Pro Ser Arg Phe Ser Gly Ser Arg Ser Gly Thr Asp Phe Thr Leu Thr
625                 630                 635                 640

Ile Ser Ser Leu Gln Pro Glu Asp Phe Ala Thr Tyr Tyr Cys Gln Gln
                645                 650                 655

His Tyr Thr Thr Pro Pro Thr Phe Gly Gln Gly Thr Lys Val Glu Ile
            660                 665                 670

Lys Ser Gly Ser Gly Ser Ser Cys Leu Ser Tyr Glu Thr Glu Ile Leu
        675                 680                 685

Thr Val Glu Tyr Gly Leu Leu Pro Ile Gly Lys Ile Val Glu Lys Arg
    690                 695                 700

Ile Glu Cys Thr Val Tyr Ser Val Asp Asn Asn Gly Asn Ile Tyr Thr
705                 710                 715                 720

Gln Pro Val Ala Gln Trp His Asp Arg Gly Glu Gln Glu Val Phe Glu
                725                 730                 735

Tyr Cys Leu Glu Asp Gly Ser Leu Ile Arg Ala Thr Lys Asp His Lys
            740                 745                 750

Phe Met Thr Val Asp Gly Gln Met Leu Pro Ile Asp Glu Ile Phe Glu
        755                 760                 765

Arg Glu Leu Asp Leu Met Arg Val Asp Asn Leu Pro Asn
    770                 775                 780

<210>  11
<211>  262
<212>  PRT
<213>  Artificial Sequence

<220>
<223>  Cyclic Tras-scFv cyclized by amide bond formation between N and C
       terminals

<400>  11

Cys Phe Asn Gly Thr His His His His His His Ala Ala Ala Glu Val
1               5                   10                  15

Gln Leu Val Glu Ser Gly Gly Gly Leu Val Gln Pro Gly Gly Ser Leu
            20                  25                  30

Arg Leu Ser Cys Ala Ala Ser Gly Phe Asn Ile Lys Asp Thr Tyr Ile
        35                  40                  45

His Trp Val Arg Gln Ala Pro Gly Lys Gly Leu Glu Trp Val Ala Arg
    50                  55                  60

Ile Tyr Pro Thr Asn Gly Tyr Thr Arg Tyr Ala Asp Ser Val Lys Gly
65                  70                  75                  80

Arg Phe Thr Ile Ser Ala Asp Thr Ser Lys Asn Thr Ala Tyr Leu Gln
                85                  90                  95

Met Asn Ser Leu Arg Ala Glu Asp Thr Ala Val Tyr Tyr Cys Ser Arg
            100                 105                 110

Trp Gly Gly Asp Gly Phe Tyr Ala Met Asp Tyr Trp Gly Gln Gly Thr
        115                 120                 125

Leu Val Thr Val Ser Ser Gly Gly Gly Gly Ser Gly Gly Gly Gly Ser
    130                 135                 140

Gly Gly Gly Gly Ser Asp Ile Gln Met Thr Gln Ser Pro Ser Ser Leu
145                 150                 155                 160

Ser Ala Ser Val Gly Asp Arg Val Thr Ile Thr Cys Arg Ala Ser Gln
                165                 170                 175

Asp Val Asn Thr Ala Val Ala Trp Tyr Gln Gln Lys Pro Gly Lys Ala
            180                 185                 190

Pro Lys Leu Leu Ile Tyr Ser Ala Ser Phe Leu Tyr Ser Gly Val Pro
        195                 200                 205

Ser Arg Phe Ser Gly Ser Arg Ser Gly Thr Asp Phe Thr Leu Thr Ile
    210                 215                 220

Ser Ser Leu Gln Pro Glu Asp Phe Ala Thr Tyr Tyr Cys Gln Gln His
225                 230                 235                 240

Tyr Thr Thr Pro Pro Thr Phe Gly Gln Gly Thr Lys Val Glu Ile Lys
                245                 250                 255

Ser Gly Ser Gly Ser Ser
            260

<210>  12
<211>  2346
<212>  DNA
<213>  Artificial Sequence

<220>

<223>  Coding substrate of cyclization

<400>  12
atggcaaaaa ctgaagaagg taaactggta atctggatta acggcgataa aggctataac      60

ggtctcgctg aagtcggtaa gaaattcgag aaagataccg gaattaaagt caccgttgag     120

catccggata aactggaaga gaaattccca caggttgcgg caactggcga tggccctgac     180

attatcttct gggcacacga ccgctttggt ggctacgctc aatctggcct gttggctgaa     240

atcaccccgg acaaagcgtt ccaggacaag ctgtatccgt ttacctggga tgccgtacgt     300

tacaacggca agctgattgc ttacccgatc gctgttgaag cgttatcgct gatttataac     360

aaagatctgc tgccgaaccc gccaaaaacc tgggaagaga tcccggcgct ggataaagaa     420

ctgaaagcga aaggtaagag cgcgctgatg ttcaacctgc aagaaccgta cttcacctgg     480

ccgctgattg ctgctgacgg gggttatgcg ttcaagtatg aaaacggcaa gtacgacatt     540

aaagacgtgg gcgtggataa cgctggcgcg aaagcgggtc tgaccttcct ggttgacctg     600

attaaaaaca aacacatgaa tgcagacacc gattactcca tcgcagaagc tgcctttaat     660

aaaggcgaaa cagcgatgac catcaacggc ccgtgggcat ggtccaacat cgacaccagc     720

aaagtgaatt atggtgtaac ggtactgccg accttcaagg gtcaaccatc caaaccgttc     780

gttggcgtgc tgagcgcagg tattaacgcc gccagtccga acaaagagct ggcaaaagag     840

ttcctcgaaa actatctgct gactgatgaa ggtctggaag cggttaataa agacaaaccg     900

ctgggtgccg tagcgctgaa gtcttacgag gaagagttgg cgaaagatcc acgtattgcc     960

gccaccatgg aaaacgccca gaaaggtgaa atcatgccga acatcccgca gatgtccgct    1020

ttctggtatg ccgtgcgtac tgcggtgatc aacgccgcca gcggtcgtca gactgtcgat    1080

gaagccctga aagacgcgca gactaattcc agctcgctgg aagttctgtt ccaggggccc    1140

catatgatca aaatagccac acgtaaatat ttaggcaaac aaaatgtcta tgacattgga    1200

gttgagcgcg accataattt tgcactcaaa aatggcttca tagcttctaa ttgtttcaat    1260

ggtacccatc atcatcacca ccacgcggcc gcagaagtgc agctggttga aagcggtggc    1320

ggtctggtgc aaccgggcgg tagcctgcgt ctgagctgcg cggcgagcgg ttttaacatc    1380

aaagacacct acattcactg ggttcgtcaa gcgccgggca agggtctgga gtgggttgcg    1440

cgtatctatc cgaccaacgg ctacacccgt tatgcggaca gcgtgaaagg tcgttttacc    1500

attagcgcgg ataccagcaa gaacaccgcg tacctgcaga tgaacagcct gcgtgcggaa    1560

gacaccgcgg tttactattg cagccgttgg ggcggtgacg gcttctacgc gatggattat    1620

tggggccaag gcaccctggt gaccgttagc agcggcggtg gaggtagtgg tggtggaggt    1680

tccggtggtg gcggtagcga tatccagatg acccagagcc cgagcagcct gagcgcgagc    1740

gtgggtgacc gtgttaccat tacctgccgt gcgagccaag atgtgaacac cgcggttgcg    1800

tggtatcagc aaaagccggg caaagcgccg aagctgctga tctacagcgc gagcttcctg 1860

tatagcggtg tgccgagccg ttttagcggc agccgtagcg gcaccgactt caccctgacc 1920

attagcagcc tgcagccgga ggattttgcg acctactatt gccagcaaca ctataccacc 1980

ccgccgacct ttggtcaagg caccaaagtt gaaatcaaga gcggatccgg gagctcgtgt 2040

ttaagctatg aaacggaaat attgacagta gaatatggat tattaccgat tggtaaaatt 2100

gtagaaaagc gcatcgaatg tactgtttat agcgttgata ataatggaaa tatttataca 2160

caacctgtag cacaatggca cgatcgcgga gaacaagagg tgtttgagta ttgtttggaa 2220

gatggttcat tgattcgggc aacaaaagac cataagttta tgactgttga tggtcaaatg 2280

ttgccaattg atgaaatatt tgaacgtgaa ttggatttga tgcgggttga taatttgccg 2340

aattga 2346

<210> 13
<211> 108
<212> PRT
<213> Artificial Sequence

<220>
<223> FKBP12

<400> 13

Met Gly Val Gln Val Glu Thr Ile Ser Pro Gly Asp Gly Arg Thr Phe
1               5                   10                  15

Pro Lys Arg Gly Gln Thr Cys Val Val His Tyr Thr Gly Met Leu Glu
            20                  25                  30

Asp Gly Lys Lys Phe Asp Ser Ser Arg Asp Arg Asn Lys Pro Phe Lys
        35                  40                  45

Phe Met Leu Gly Lys Gln Glu Val Ile Arg Gly Trp Glu Glu Gly Val
    50                  55                  60

Ala Gln Met Ser Val Gly Gln Arg Ala Lys Leu Thr Ile Ser Pro Asp
65                  70                  75                  80

Tyr Ala Tyr Gly Ala Thr Gly His Pro Gly Ile Ile Pro Pro His Ala
                85                  90                  95

Thr Leu Val Phe Asp Val Glu Leu Leu Lys Leu Glu
            100                 105

<210> 14
<211> 327
<212> DNA
<213> Artificial Sequence

<220>
<223>  coding FKBP12

<400>  14
atgggagtgc aggtggaaac catctcccca ggagacgggc gcaccttccc caagcgcggc      60

cagacctgcg tggtgcacta caccgggatg cttgaagatg gaaagaaatt tgattcctcc     120

cgggacagaa acaagccctt taagtttatg ctaggcaagc aggaggtgat ccgaggctgg     180

gaagaagggg ttgcccagat gagtgtgggt cagagagcca aactgactat atctccagat     240

tatgcctatg gtgccactgg gcacccaggc atcatcccac cacatgccac tctcgtcttc     300

gatgtggagc ttctaaaact ggaatga                                         327


<210>  15
<211>  102
<212>  PRT
<213>  Artificial Sequence

<220>
<223>  DnaE-N

<400>  15

Cys Leu Ser Tyr Glu Thr Glu Ile Leu Thr Val Glu Tyr Gly Leu Leu
1               5                   10                  15


Pro Ile Gly Lys Ile Val Glu Lys Arg Ile Glu Cys Thr Val Tyr Ser
            20                  25                  30


Val Asp Asn Asn Gly Asn Ile Tyr Thr Gln Pro Val Ala Gln Trp His
        35                  40                  45


Asp Arg Gly Glu Gln Glu Val Phe Glu Tyr Cys Leu Glu Asp Gly Ser
    50                  55                  60


Leu Ile Arg Ala Thr Lys Asp His Lys Phe Met Thr Val Asp Gly Gln
65                  70                  75                  80


Met Leu Pro Ile Asp Glu Ile Phe Glu Arg Glu Leu Asp Leu Met Arg
                85                  90                  95


Val Asp Asn Leu Pro Asn
            100


<210>  16
<211>  35
<212>  PRT
<213>  Artificial Sequence

<220>
<223>  DnaE-C

<400>  16

Ile Lys Ile Ala Thr Arg Lys Tyr Leu Gly Lys Gln Asn Val Tyr Asp
1               5                   10                  15

Ile Gly Val Glu Arg Asp His Asn Phe Ala Leu Lys Asn Gly Phe Ile
            20                  25                  30

Ala Ser Asn
        35

## Description

### TECHNICAL FIELD

The present invention relates to a new single-chain antibody and a production method thereof.

### BACKGROUND ART

Monoclonal antibodies are used as therapeutic substances at various clinical sites including oncology, chronic inflammatory disease, transplant, infectious disease, cardiovascular internal medicine, or ophthalmologic disease, and is further used for various uses such as diagnostic drugs and sensor elements. The main function of an antibody is specifically binding to an antigen (target molecule), and an antigen is recognized by an Fv domain.

An Fv domain of an antibody comprises an Fv domain derived from a heavy chain (VH) and an Fv domain derived from a light chain (VL). Even in Fv fragments which are fragments cut out of antibodies, many antibodies maintain the ability to bind to targets, and Fv fragments constitute the smallest units of the antigen-binding function of antibodies. A single-chain antibody (scFv: single-chain Fv) is an antibody in which a VH and a VL bind with a peptide linker. For example, an scFv which recognizes an advanced glycation end product (AGE) is reported (NPL 1).

Cells such as CHO cells and hybridomas derived from eukaryotes are generally used to produce monoclonal antibodies, and the production requires great cost. The molecular weight of an scFv is around 25 kDa, and the molecular weight thereof is remarkably small as compared with a full-length antibody. Therefore, production using a procaryote such as *Escherichia coli* as a host is possible, and an scFv has an advantage in production cost as compared with a full-length antibody.

A cyclic single-chain trispecific antibody corresponding to the trimer of an scFv is reported (PTL 1). Meanwhile, an scFv generally has the characteristic of associating and forming a polymer, and dimers, trimers and tetramers which scFvs form are reported (NPLS 2 to 4). The characteristic of scFvs thus forming polymers easily was a problem in the viewpoint of improvement in the stability of scFvs.

The stability of antibody molecules includes three meanings. A first is thermal resistance or thermal stability, a second is the stability to proteases, and a third is storage stability by suppressing the formation of associated bodies. An scFvs had a problem especially with storage stability.

A method using sortase in the modification of a peptide is reported (PTLS 2 and 3 and NPLS 5 and 6), and a method for synthesizing a cyclic peptide using sortase is also reported (NPL 7).

An intein comprises a central intein and sequences between which it is located (exteins). An intein is an internal protein factor which catalyzes the reaction of self-excising an intein portion from a host sequence and linking sequences between which the intein is located (exteins) by a peptide bond. An intein reaction is a post-translational process which does not require an auxiliary enzyme or a cofactor (NPL 8). An extein sequence disposed upstream of an intein is called "N-extein", and an extein disposed downstream of the intein is called "C-extein." As products of an intein reaction, two stable proteins which are a mature protein and an intein are obtained.

An intein can be divided into two, and can exist as two fragments encoded by two genes transcribed and translated individually. A divided intein is called a split intein, self-associates and catalyzes the protein splicing reaction of linking an N-extein and a C-extein by a peptide bond. A split intein can be produced by dividing an intein artificially. Split inteins also exist in nature, and are confirmed in various cyanobacteria and archaebacteria (NPLS 9 to 14). DnaE derived from *Nostoc punctiforme* (*Npu*DnaE) is a split intein which exhibits the highest intein reaction efficiency of split inteins reported in NON PATENT LITERATURES (NPLS 14 and 15). The use of an intein reaction as the cyclization reaction of a protein of interest is reported (PTLS 4 and 5).

### CITATION LIST

### PATENT LITERATURE

PTL 1: JP 2005-501517 A
PTL 2: JP 2015-527981 A
PTL 3: JP 2015-527982 A
PTL 4: US 2011/321183 A1
PTL 5: WO 2016/167291 A1

### NON PATENT LITERATURE

NPL 1: Molecules. 2017 22:e1695;
NPL 2 : Proc. Natl. Acad. Sci. USA. 1997, 94, 9637-9642;
NPL 3 : Journal of Immunological Methods 248 (2001) 47-66;
NPL 4 : FEBS Letters 425 (1998) 479-484;
NPL 5 : Journal of Biotechnology 152 (2011) 37-42;
NPL 6 : Langmuir 2012, 28, 3553-3557;
NPL 7 : Biol.Chem. 2015 396:283-293.
NPL 8 : Perler F et al., Nucl Acids Res. 22:1125-1127 (1994)
NPL 9 : Caspi et al., Mol Microbiol. 50:1569-1577 (2003);
NPL 10 : Choi J et al., J Mol Biol. 356:1093-1106 (2006);
NPL 11 : Dassa B et al., Biochemistry. 46:322-330 (2007);
NPL 12 : Liu X and Yang J., J Biol Chem 278:26315-26318 (2003);
NPL 13 : WU H et al., Proc Natl Acad Sci USA. 95:9226-9231 (1998);
NPL 14 : Zettler J. et al., FEBS Letters. 583:909-914 (2009)
NPL 15 : Iwai H et al., FEBS Letters 580:1853-1858 (2006)

### SUMMARY OF INVENTION

### TECHNICAL PROBLEM

An antibody can have a closed state in which a VH region and a VL region associate and an open state in which the association of both loosens. Generally, a VH region and a VL region are easily in the closed state due to the existence of the Fc region in a full-length antibody as compared with an scFv. Meanwhile, an scFv is easily in an open structure as compared with a full-length antibody. A VH region and a VL region have the characteristic of forming an associated body by interaction. In scFvs, when the rate of the states in which VH regions and VL regions are open increases, an associated body of a VH and a VL is not only formed in a molecule, but an associated body of a VH and a VL between molecules is also formed. Therefore, scFvs easily form oligomer. When the association further proceeds, aggregates are produced. ScFvs easily caused the ununiformity of molecular size, and such instability prevented the industrial use of scFvs.

The industrial use was limited due to a problem with stability in conventional scFvs. An object of the present invention is to solve such a problem and provide a technique for promoting the industrial use of scFvs.

### SOLUTION TO PROBLEM

Mutagenesis has been performed as a method for stabilizing scFvs conventionally. Mutagenesis is a method based on trial and error, many mutants need to be produced, and the stabilities need to be examined one by one. However, since mutation sites and the types of replacement amino acids relating to stabilization vary depending on the antibody, each antibody needs to be optimized.

The present inventors have found that cyclizing an scFv enables stabilizing the scFv and especially suppressing the formation of associated bodies and completed the present invention. That is, the present invention relates to a cyclic scFv wherein the formation of associated bodies is suppressed, and the storage stability is improved and a production method thereof.

The present invention provides the following inventions.
[A-1] A single-chain antibody (scFv) comprising a heavy chain variable region (VH) and a light chain variable region (VL) linked by a first peptide linker, wherein an N-terminus and a C-terminus thereof are linked by a second peptide linker to form a cyclic single-chain antibody.
[A-2] The single-chain antibody according to [A-1], wherein the second peptide linker is formed by transpeptidase.
[A-3] The single-chain antibody according to [A-2], wherein the transpeptidase is sortase.
[A-4] The single-chain antibody according to any of [A-1] to [A-3], wherein the second peptide linker consists of an amino acid sequence: LPXTG wherein X represents any amino acid residue.
[A-5] The single-chain antibody according to any of [A-1] to [A-4], wherein the first peptide linker consists of 15 to 27 amino acids.
[A-6] The single-chain antibody according to any of [A-1] to [A-5], wherein the second peptide linker consists of 19 to 28 amino acids.
[A-7] The single-chain antibody according to any one of [A-1] to [A-6], wherein cohesiveness at the time of storage is suppressed as compared with an acyclic single-chain antibody having the same heavy chain variable region (VH) and the same light chain variable region (VL).
[A-8] A method for producing the cyclic single-chain antibody according to any of [A-1] to [A-7], comprising the steps of:
   1) preparing an acyclic single-chain antibody which is a single-chain antibody (scFv) comprising a heavy chain variable region (VH) and a light chain variable region (VL) linked by a first peptide linker, and has transpeptidase recognition sequences at the N-terminus and the C-terminus; and
   2) forming a second peptide linker from the transpeptidase recognition sequences of the N-terminus and the C-terminus of the single-chain antibody using transpeptidase and cyclizing the single-chain antibody.
[A-9] The production method according to [A-8], wherein the transpeptidase is sortase.
[A-10] The production method according to [A-8] or [A-9], wherein the transpeptidase recognition sequence of the N-terminus of the acyclic single-chain antibody comprises LPXTG wherein X represents any amino acid residue.
[A-11] The production method according to any of [A-8] to [A-10], wherein the transpeptidase recognition sequence of the C-terminus of the acyclic single-chain antibody comprises GG.
[B-1] A single-chain antibody (scFv) comprising a heavy chain variable region (VH) and a light chain variable region (VL) linked by a first peptide linker, wherein an N-terminus and a C-terminus of the single-chain antibody are linked by a second peptide linker to form a cyclic single-chain antibody.
[B-2] The single-chain antibody according to [B-1], wherein the single-chain antibody has a cyclic structure formed by only peptide bonds.
[B-3] The single-chain antibody according to [B-1] or [B-2], wherein the VH and the VL are associated in a molecule, and form an antigen-binding site.
[B-4] The single-chain antibody according to any of [B-1] to [B-3], wherein the cyclic single-chain antibody has one antigen-binding site in a molecule.
[B-5] The single-chain antibody according to any of [B-1] to [B-4], wherein the first peptide linker consists of 15 to 27 amino acids.
[B-6] The single-chain antibody according to any of [B-1] to [B-5], wherein the second peptide linker consists of 15 to 28 amino acids.
[B-7] The single-chain antibody according to any of [B-1] to [B-6], wherein aggregate formation is suppressed as compared with an acyclic single-chain antibody having the same VH and the same VL.
[B-8] The single-chain antibody according to any of [B-1] to [B-7], wherein the second peptide linker is formed by transpeptidase.
[B-9] The single-chain antibody according to [B-8], wherein the transpeptidase is sortase.
[B-10] The single-chain antibody according to any of [B-1] to [B-9], wherein the second peptide linker comprises an amino acid sequence: LPXTG wherein X represents any amino acid residue.
[B-11] The single-chain antibody according to any of [B-1] to [B-7], wherein the second peptide linker is formed by trans-splicing reaction by a split intein.
[B-12] A method for producing the cyclic single-chain antibody according to any of [B-1] to [B-7], comprising the steps of:
   1) preparing an acyclic peptide which is a single-chain antibody (scFv) comprising a heavy chain variable region (VH) and a light chain variable region (VL) linked by a first peptide linker, and has transpeptidase recognition sequences at the N-terminus and the C-terminus; and
   2) forming a second peptide linker from the transpeptidase recognition sequences of the N-terminus and the C-terminus of the single-chain antibody using transpeptidase and cyclizing the single-chain antibody.
[B-13] The production method according to [B-8], wherein the transpeptidase is sortase.
[B-14] The production method according to [B-12] or [B-13], wherein the transpeptidase recognition sequence of the N-terminus of the acyclic peptide comprises LPXTG wherein X represents any amino acid residue.
[B-15] The production method according to any of [B-12] to [B-14], wherein the transpeptidase recognition sequence of the C-terminus of the acyclic peptide comprises GG.
[B-16] A method for producing the cyclic single-chain antibody according to any of [B-1] to [B-7], comprising the steps of:
   1) preparing an acyclic peptide which is a single-chain antibody (scFv) comprising a heavy chain variable region (VH) and a light chain variable region (VL) linked by the first peptide linker, and has a C-terminal split intein fragment (Int-C) at the N-terminus and an N-terminal split intein fragment (Int-N) at the C-terminus; and
   2) forming a second peptide linker by trans-splicing reaction by the split intein and cyclizing the single-chain antibody.
[B-17] The production method according to [B-16], wherein the C-terminal split intein fragment (Int-C) used is DnaE-Int-C, and the N-terminal split intein fragment (Int-N) used is DnaE-Int-N.
[B-18] A nucleic acid comprising a nucleotide sequence encoding an amino acid sequence of the acyclic peptide according to step 1 of [B-16].
[B-19] A recombinant vector comprising the nucleic acid according to [B-18].
[B-20] A transformant comprising the recombinant vector according to [B-19] introduced thereinto.

### ADVANTAGEOUS EFFECTS OF INVENTION

The present invention relates to a method for suppressing the association property between molecules and improving storage stability by cyclizing an scFv. The method of the present invention is highly versatile, and can be commonly used for single-chain antibodies. Since the terminal residues do not exist in the cyclic scFv according to the present invention, the cyclic scFv is hardly hydrolyzed by exopeptidase, which is an enzyme which hydrolyzes a polypeptide from the N-terminus or the C-terminus. The stability of the scFv can be improved, and the use of the scFv can be promoted by the method of the present invention. The application to the production of a stable scFv medically or industrially useful for the development of sensors using the scFv as sensor elements, drugs, the artificial control of receptor signals, and the like can be expected.

### BRIEF DESCRIPTION OF DRAWINGS

[Fig. 1] Fig. 1 shows a schematic diagram of a cyclic single-chain antibody.
[Fig. 2] Fig. 2 shows the domain structure of a polypeptide (Y9-scFv-LPETG) to be subjected to cyclization reaction by sortase.
[Fig. 3] Fig. 3 shows the amino acid sequence of a polypeptide (Y9-scFv-LPETG) (SEQ ID NO: 1) to be subjected to cyclization reaction by sortase.
[Fig. 4] Fig. 4 shows a schematic illustration of the periphery of a region encoding Y9-scFv of pSAL-Y9-scFv.
[Fig. 5] Fig. 5 shows the result of SDS-PAGE of cyclic Y9-scFv subjected to cyclization reaction using sortase A and purified.
[Fig. 6A] Fig. 6A shows the distribution of the hydrodynamic radius determined from dynamic light scattering. □ indicates the distribution of the hydrodynamic radius of acyclic Y9-scFv 1 day after preparation, and ▲ indicates the distribution of the hydrodynamic radius of acyclic Y9-scFv 14 days after preparation.
[Fig. 6B] Fig. 6B shows the distribution of the hydrodynamic radius determined from dynamic light scattering. □ indicates the distribution of the hydrodynamic radius of cyclic Y9-scFv 1 day after preparation cyclized by a sortase, and ▲ indicates the distribution of the hydrodynamic radius of cyclic Y9-scFv 14 days after preparation cyclized by a sortase.
[Fig. 7] Fig. 7 shows the comparison of the antigen affinities of cyclic Y9-scFv cyclized by sortase and acyclic Y9-scFv by surface plasmon resonance (SPR). □ indicates a datum of cyclic Y9-scFv cyclized by sortase, and ▲ indicates a datum of acyclic Y9-scFv.
[Fig. 8] Fig. 8 is a figure showing the comparison of thermal denaturation curves by differential scanning fluorescence measurement. □ indicates a datum of the differential scanning fluorescence measurement of cyclic Y9-scFv cyclized by sortase, and ▲ indicates a datum of the differential scanning fluorescence measurement of acyclic Y9-scFv.
[Fig. 9] Fig. 9 shows the sequence of the encoding region of a plasmid (pSAL-Y9-scFv) (SEQ ID NO: 2).
[Fig. 10] Fig. 10 shows a schematic diagram of the cyclization of an scFv by an intein reaction.
[Fig. 11] Fig. 11 shows a schematic diagram of the structure of acyclic Tras-scFv used as a comparison object.
[Fig. 12] Fig. 12 shows the amino acid sequence of a polypeptide (acyclic Tras-scFv) (SEQ ID NO: 3).
[Fig. 13] Fig. 13 shows a schematic illustration of the periphery of a region encoding acyclic Tras-scFv of pET28-Tras-scFv.
[Fig. 14] Fig. 14 shows the result of SDS-PAGE of purified acyclic Tras-scFv.
[Fig. 15] Fig. 15 shows a schematic diagram of the structure of a polypeptide (Tras-scFv-LPETG) to be subjected to cyclization reaction by sortase.
[Fig. 16] Fig. 16 shows the amino acid sequence of a polypeptide (Tras-scFv-LPETG) (SEQ ID NO: 5) to be subjected to cyclization reaction by sortase.
[Fig. 17] Fig. 17 shows a schematic illustration of the periphery of a region encoding Tras-scFv-LPETG of pET28-Tras-scFv-LPETG.
[Fig. 18] Fig. 18 shows the result of SDS-PAGE of cyclic Tras-scFv subjected to cyclization reaction using sortase A and purified.
[Fig. 19] Fig. 19 shows the distribution of the hydrodynamic radius determined from dynamic light scattering. □ indicates the distribution of the hydrodynamic radius of acyclic Y9-scFv 1 day after preparation, and ■ indicates the distribution of the hydrodynamic radius of cyclic Y9-scFv 1 day after preparation cyclized by sortase.
[Fig. 20] Fig. 20 shows a schematic diagram of the structure of GST-HER2, which is used for the evaluation of the antigen-binding activity of acyclic Tras-scFv and cyclic Tras-scFv cyclized by sortase.
[Fig. 21] Fig. 21 shows the amino acid sequence of a polypeptide (GST-HER2) (SEQ ID NO: 8).
[Fig. 22] Fig. 22 shows the result of SDS-PAGE of a fused protein (GST-HER2) of a peptide fragment containing a trastuzumab-binding region of HER2 (HER2-Tras-binding fragment) and glutathione S-transferase (GST).
[Fig. 23] Fig. 23 shows the comparison of the antigen affinities of cyclic Tras-scFv cyclized by sortase and acyclic Tras-scFv by surface plasmon resonance (SPR). The solid line indicates the data of cyclic Tras-scFv cyclized by sortase, and the broken line indicates the data of acyclic Tras-scFv.
[Fig. 24] Fig. 24 shows a schematic diagram of the structure of a polypeptide (Tras-scFv-Intein) to be subjected to cyclization reaction by an intein.
[Fig. 25] Fig. 25 shows the amino acid sequence of a polypeptide (Tras-scFv-Intein) (SEQ ID NO: 10) to be subjected to cyclization reaction by an intein.
[Fig. 26] Fig. 26 shows a schematic illustration of the periphery of a region encoding Tras-scFv-Intein of pNMK-Tras-scFv.
[Fig. 27] Fig. 27 shows the amino acid sequence of a polypeptide (FKBP12) (SEQ ID NO: 13) used for the expression of a polypeptide (Tras-scFv-Intein) to be subjected to cyclization reaction by an intein.
[Fig. 28] Fig. 28 shows a schematic illustration of the periphery of a region encoding FKBP12 of pET21-FKBP12.
[Fig. 29] Fig. 29 shows the result of SDS-PAGE of cyclic Tras-scFv subjected to cyclization reaction using an intein in colon bacilli and purified.
[Fig. 30] Fig. 30 shows the comparison of the antigen affinities of cyclic Tras-scFv cyclized by an intein and acyclic Tras-scFv by surface plasmon resonance (SPR). The solid line indicates the data of cyclic Tras-scFv cyclized by an intein, and the broken line indicates the data of acyclic Tras-scFv.
[Fig. 31] Fig. 31 shows the comparison of the residual activities of cyclic Tras-scFv cyclized by an intein and acyclic Tras-scFv at the time of redissolution after freeze-drying by surface plasmon resonance (SPR). The solid line indicates the data of cyclic Tras-scFv cyclized by an intein, and the broken line indicates the data of acyclic Tras-scFv.

### DESCRIPTION OF EMBODIMENTS

A cyclic scFv according to the present invention has a cyclic structure by covalent bonds. Examples of the method for producing a cyclic scFv include intein reaction, in which an intein is used, and native chemical ligation. Examples of other linking methods include a method using glutaminase and a chemical linking method.

In one embodiment of the present invention, the cyclic scFv is produced by an enzymatic linking method using transpeptidase. Examples of the transpeptidase include sortase, and a well-known sortase enzyme (Chen et al., PNAS 108: 11399-11404, 2011; Popp et al., Nat Chem Biol 3: 707-708, 2007) can be used. Preferable example of the sortase include sortase A. Soluble shortened sortase A lacking in a transmembrane region (SrtA; amino acid residues 60 to 206 of *Staphylococcus aureus* SrtA) can also be used (Ton-That, H., et al., Proc. Natl. Acad. Sci. USA 96 (1999) 12424-12429; Ilangovan, H., et al., Proc. Natl. Acad. Sci. USA 98 (2001) 6056-6061). A shortened soluble sortase A variant can be prepared in colon bacillus (*E. coli*)*.*

Sortase can be used to link the N-terminus of a protein to a position near the C-terminus of another protein by a covalent bond, and can also be used for intramolecular cyclization reaction. Sortase recognizes, for example, N-terminal GGG and C-terminal LPXTGX'n wherein X and X' are amino acids selected arbitrarily and independently, and n can be amino acids in any number including, for example, 1 to 99 (for example, natural amino acids). Sortase subsequently facilitates the rearrangement of glycine residues in two peptide sequences, produces a covalent bond between two peptide sequences, and releases GX'n. In one embodiment of the present invention, the cyclic scFv is produced by cyclizing a single-chain antibody having linking portions for enzymatic linkage using sortase at the N-terminus and the C-terminus.

Examples of the X and the X' in the above-mentioned sequence include natural amino acids, and specifically include alanine, arginine, asparagine, aspartic acid, cysteine, glutamine, glutamic acid, glycine, histidine, isoleucine, leucine, lysine, methionine, phenylalanine, serine, threonine, tryptophan, tyrosine, proline, and valine. More specific examples of the X include glutamic acid, and more specific examples of the X' include leucine.

A VH and a VL comprise around 100 to 120 amino acid residues called "immunoglobulin fold", have the same overall form, and mainly comprise β-sheets. An acyclic scFv used for preparing a cyclic scFv has glycine at the N-terminus, and has LPXTG (X is any amino acid residue) in a C-terminus region. In the acyclic scFv, the VH and the VL may be located sequentially, and the VL and the VH may be located sequentially from the N-terminus.

Regions corresponding to a VH and a VL in an antibody can be determined based on well-known knowledge. Literatures such as Martin, A.C.R. Accessing the Kabat Antibody Sequence Database by Computer PROTEINS: Structure, Function and Genetics, 25 (1996), 130-133; and Elvin A. Kabat, Tai Te Wu, Carl Foeller, Harold M. Perry, Kay S. Gottesman (1991) Sequences of Proteins of Immunological Interest can be consulted advantageously. In one aspect of the present invention, the VH region is defined as the amino acid sequences of SEQ ID NOS: 0 to 113 of Kabat, and the VL region is defined as the amino acid sequences of SEQ ID NOS: 0 to 109 of Kabat. In one embodiment, as to an antibody in which 2 to 3 residues on the C-terminus side of the framework 4 do not form secondary structure in both of the VH and the VL, regions not containing the residues can be defined as a VH or a VL.

β-Sheets exist near the terminals of the VH and VL regions. In the acyclic scFv used for producing the cyclic scFv, the number of amino acid residues between the linking portion (oligoglycine Gₘ and LPXTG) and the nearest β-sheet on the sequence is preferably 3 or more, and more preferably 5 or more. As to the Gly at the N-terminus, the number of amino acid residues from the nearest β-sheet on the sequence is preferably 3 or more, and more preferably 5 or more.

In one embodiment of the present invention, intein reaction, which is the splicing reaction of proteins, more specifically trans-splicing reaction using a split intein can be utilized for producing the cyclic scFv.

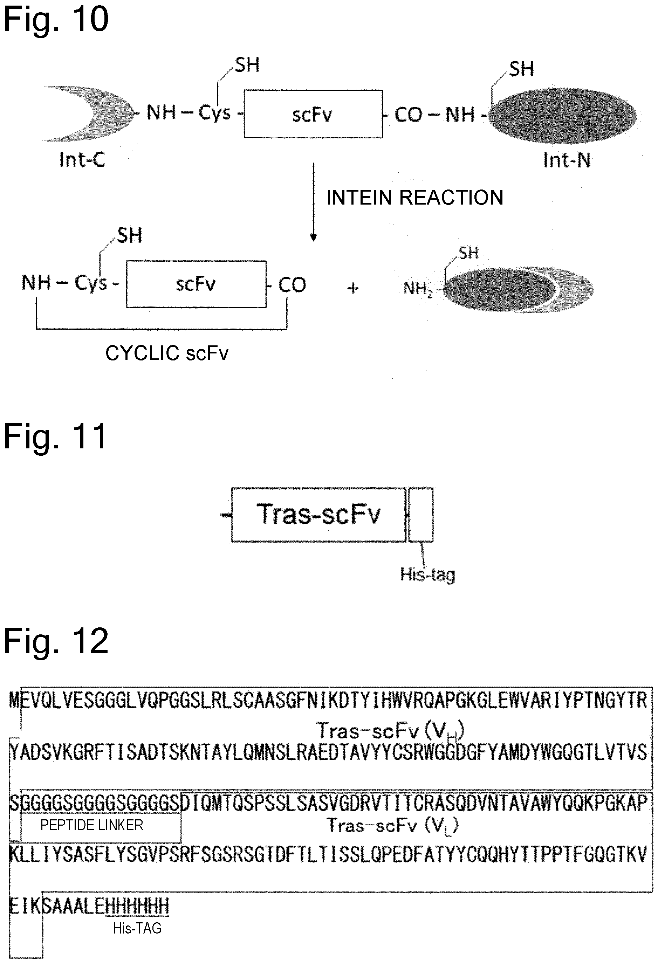

Among two into which the intein is divided, a fragment on the N-terminus side is referred to as an Int-N, and a fragment on the C-terminus side is referred to as an Int-C. In one embodiment, the cyclic single-chain antibody of the present invention can be produced by preparing a protein in which the N-terminus of a protein to be cyclized is fused with the C-terminus of the Int-C, and the N-terminus of the Int-N is further fused with the C-terminus side thereof (Fig. 10). To obtain the cyclic scFv, a polypeptide chain in which the N-terminus of the acyclic scFv is fused with the C-terminus of the Int-C, and the N-terminus of the Int-N is linked with the C-terminus of the acyclic scFv is specifically provided, and cyclization reaction can be performed spontaneously. In the present invention, cyclization reaction in cells using an intein protein can be used to cyclize a protein. DnaE derived from *Nostoc punctiforme* (*Npu*DnaE) can be used for an intein protein, and DnaE-Int-N (DnaE-N) and DnaE-Int-C(DnaE-C), which are proteins comprising amino acid sequences represented by SEQ ID NO: 15 and SEQ ID NO: 16, can be more specifically used.

In the present invention, DnaE derived from *Nostoc punctiforme* can be used as an intein protein for cyclizing a protein. DnaE-N and DnaE-C which are proteins comprising amino acid sequences represented by SEQ ID NO: 15 and SEQ ID NO: 16 can be more specifically used. In the polypeptide chain in which the protein of interest is linked between these DnaE-C and DnaE-N, splicing proceeds spontaneously by the self-catalytic ability of DnaE, and a cyclic protein in which a peptide bond (amide bond) is formed between the amino group at the N-terminus of the protein of interest and the carbonyl group at the C-terminus is synthesized. The above-mentioned cyclization reaction can also be performed in cells by expressing a polypeptide which is the substrate of the reaction in cells.

In one embodiment, when a split intein is used, a polypeptide which is the substrate of cyclization reaction has cysteine or serine on either the amino group side or the carboxylic acid side of a portion at which a peptide bond (amide bond) for cyclization is produced, and the residue on the carboxylic acid side is a residue other than proline.

The second peptide linker formed by cyclization using the split intein may contain an amino acid sequence selected, for example, from CFNGT, CFN, CYNGT, or CYN. In one embodiment, the amino groups of the Cs of these amino acid sequences form a peptide bond (amide bond) for cyclization.

The second peptide linker may contain the amino acid sequence GSGSS. In one embodiment, the carboxyl group of an S of the amino acid sequence forms a peptide bond (amide bond) for cyclization.

In the acyclic scFv to be used for producing the cyclic scFv, the heavy chain variable region (VH) and the light chain variable region (VL) are linked by the first peptide linker. In one embodiment of the present invention, the number of the amino acid residues of the first peptide linker is, for example, 10 or more, 13 or more, or 15 or more, and is 27 or less, 25 or less, or 23 or less. Amino acids constituting the first peptide linker are not particularly limited as long as the amino acids are natural amino acids. Examples of the amino acid include glycine, alanine, valine, isoleucine, leucine, serine, threonine, cysteine, methionine, phenylalanine, tryptophan, tyrosine, proline, glutamic acid, aspartic acid, glutamine, asparagine, lysine, arginine, and histidine, and specifically include glycine, alanine, serine, phenylalanine, glutamic acid, and arginine. The number of the residues of the first peptide linker affects improvement in the proceeding of cyclization reaction and the association characteristic.

The cyclic single-chain antibody of the present invention has the characteristic of the cyclic structure being formed by only peptide bonds. The above-mentioned cyclic structure may further have the crosslinked structure by -SS- bonds. In one embodiment of the present invention, the heavy chain variable region (VH) and the light chain variable region (VL) forming ring structure associate, and form an antigen-binding site in the cyclic single-chain antibody. The cyclic single-chain antibody of the present invention has one antigen binding site which the VH and the VL associate and form in a molecule.

The second peptide linker is formed between the heavy chain variable region (VH) and the light chain variable region (VL) by the cyclization of the acyclic scFv to be used for producing the cyclic scFv. In one embodiment of the present invention, the number of the amino acid residues of the second peptide linker is, for example, 12 or more, 13 or more, 15 or more, 16 or more, 19 or more, or 21 or more, and is 28 or less, 26 or less, and 24 or less. Amino acids constituting the second peptide linker are not particularly limited as long as the amino acids are natural amino acids. Examples of the amino acids include glycine, alanine, valine, isoleucine, leucine, serine, threonine, cysteine, methionine, phenylalanine, tryptophan, tyrosine, proline, glutamic acid, aspartic acid, glutamine, asparagine, lysine, arginine, and histidine, and specifically include glycine, alanine, serine, phenylalanine, glutamic acid, and arginine. The number of the residues of the second peptide linker affects improvement in the proceeding of cyclization reaction and the association characteristic.

Although the method for producing the cyclic scFv of the present invention is not particularly limited, the following method is illustrated.
(1) Glycine is introduced into the N-terminus of an scFv. Amino peptidase which inheres in an expressing host is used for preparing a protein having glycine at the N-terminus. Inserting the cleavage sequence of protease such as the recognition sequence of Tev protease (ENLYFQ/G wherein / in the recognition sequence represents a cleavage site) and the recognition sequence of HRV3C protease (LEVLFQ/GG or LEVLFQ/GP wherein / in the recognition sequence represents a cleavage site) upstream of glycine enables preparing the scFv having glycine at the N-terminus by digestion with protease.
(2) Making sortase A act on the scFv prepared by the procedure illustrated by (1), having glycine at the N-terminus, and containing the LPXTG sequence at the C-terminus enables preparing a cyclic scFv. The linking reaction is performed in an almost neutral buffer solution (pH 5.5 to 9.5). Calcium ions need to be further added to the reaction liquid depending on sortase A to be used.
(3) All the hosts including colon bacilli, yeast, mammalian cells, insect cells, and the like can be used for preparing an scFv to be used for preparing a cyclic scFv, and all the promotors including T7, Taq, lac, and the like can be used as promotors.

A peptide which is the substrate of cyclization reaction may fuse with another peptide or another protein, or a fragment thereof for enhancing solubilization besides a structure required for obtaining a cyclic peptide of interest. Examples of the peptide and the protein used for fusion include myelin basic protein (MBP), thioredoxin, glutathione S-transferase (GST), and the protein GB1 domain (GB1).

The cyclization of the scFv can be detected using sodium dodecyl sulfate polyacrylamide gel electrophoresis (SDS-PAGE). When the scFv is cyclized, the mobility changes in electrophoresis. Therefore, the cyclization can be detected by comparison with the mobility of the scFv which is not cyclized. At this time, a product which is not cyclized and in which a region downstream of glycine in the LPXTG sequence is cleaved may also be produced. The following method is illustrated as a method for distinguishing this cleaved product from the cyclic scFv. Although the cyclic scFv is not digested with carboxypeptidase or aminopeptidase, the above-mentioned cleaved product is digested.

### Examples of the other methods include mass spectrometry.

The "single-chain antibody" herein means a single-chain Fv fragment (scFv) obtained by linking an Fv domain derived from a heavy chain of a full-length antibody (VH) and an Fv domain derived from a light chain (VL) by a peptide linker. As long as the peptide linker has a sequence suitable for the single-chain antibody to have an antigen-binding property, here, the peptide linker is not particularly limited, and comprises for example, 10 or more amino acids, specifically 10 to 27 amino acids, more specifically 15 to 20 amino acids. As long as amino acids constituting the peptide linker are natural amino acids, the amino acids are not particularly limited. Examples of the amino acids include glycine, alanine, valine, isoleucine, leucine, serine, threonine, cysteine, methionine, phenylalanine, tryptophan, tyrosine, proline, glutamic acid, aspartic acid, glutamine, asparagine, lysine, arginine, and histidine, and specifically include glycine, alanine, serine, phenylalanine, glutamic acid, and arginine.

The single-chain antibody may have any of a structure in which the C-terminus of the VL domain and the N-terminus of the VH domain are linked by a peptide linker and a structure in which the C-terminus of VH domain and the N-terminus of the VL domain are linked by a peptide linker.

The scFv used in the present invention can be produced by methods described in books (for example, edited by Carl A. K. Borrebaeck, (1995) Antibody Engineering (Second Edition), Oxford University Press, New York; edited by John McCafferty, Hennie Hoogenboom, Dave Chiswell, (1996) Antibody Engineering, A Practical Approach, IRL Press, Oxford; and the like) and literatures (for example, Biochim. Biophys. Acta - Protein Structure and Molecular Enzymology 1385, 17-32 (1998); Molecules. 2017 22:e1695; Journal of Biochemistry 161:37-43; and the like).

The cyclic single-chain antibody of the present invention can be produced by culturing a transformant prepared by a genetic engineering technique. A nucleic acid encoding the amino acid sequence of an acyclic peptide which is the substrate of cyclization reaction for producing the cyclic single-chain antibody can be synthesized by chemical synthesis, PCR, cassette mutagenesis, site-specific mutagenesis, or the like. For example, chemically synthesizing a plurality of oligonucleotides having a complementary region having around 20 base pairs at a terminal and comprising around 100 bases or less and performing an overlap extension method in combination of these enable synthesizing the whole of a nucleic acid of interest. The above-mentioned acyclic peptide used as the substrate of cyclization reaction may be called an acyclic single-chain antibody herein. At this time, although the acyclic peptide and the acyclic single-chain antibody contain elements such as a heavy chain variable region (VH) and a light chain variable region (VL) required as a single-chain antibody, it does not matter whether the acyclic peptide and the acyclic single-chain antibody have antigen-binding activity or not.

The recombinant vector of the present invention can be obtained by linking (insert) the above-mentioned nucleic acid with a suitable vector. As long as the vector used in the present invention is a vector which can be replicated in a host or in which a nucleic acid of interest can be incorporated into a host genome, the vector is not particularly limited. For example, bacteriophages, plasmids, cosmids, phagemids, and the like are mentioned.

Examples of the plasmid DNA include plasmids derived from actinomycetes (for example, pK4, pRK401, pRF31, and the like), plasmids derived from colon bacilli (for example, pBR322, pBR325, pUC118, pUC119, pUC18, and the like), plasmids derived from hay bacilli (for example, pUB110, pTP5, and the like), and plasmids derived from yeast (for example, YEp13, YEp24, YCp50, and the like). Examples of the phage DNA include λ phages (λgt10, λgt11, λZAP, and the like). Additionally, an animal virus such as a retrovirus or a vaccinia virus; or an insect virus vector such as a baculovirus can also be used.

To insert a gene into a vector, a method for first cleaving purified DNA with suitable restriction enzymes, inserting the gene between the restriction enzyme sites of a suitable vector DNA or into the multicloning site, and linking the gene with the vector, or the like is adopted. The gene needs to be incorporated into the vector so that the improved protein of the present invention is expressed. Then, a cis element such as an enhancer, a splicing signal, a poly A addition signal, a selection marker, a ribosome-binding sequence (SD sequence), an initiation codon, a termination codon, and the like can be optionally linked to the vector of the present invention besides a promotor and the nucleotide sequence of the gene. A tag sequence for facilitating the purification of a protein to be produced can also be linked. A nucleotide sequence encoding a well-known tag such as a His tag, a GST tag, or an MBP tag can be used as a tag sequence.

It can be confirmed using well-known genetic engineering technology whether the gene is inserted in the vector or not. For example, in the case of a plasmid vector or the like, the confirmation can be performed by subcloning the vector using competent cells, extracting the DNA, and then determining the nucleotide sequence using a DNA sequencer. The same technique can be used for other vectors which can be subcloned using bacteria or other hosts. Vector selection using a selection marker such as a drug resistance gene is also effective.

A transformant can be obtained by introducing the recombinant vector of the present invention into a host cell so that the improved protein of the present invention can be expressed. As long as the host to be used for transformation can express a protein or a polypeptide, the host is not particularly limited. For example, bacteria (colon bacilli, hay bacilli and the like), yeast, plant cells, animal cells (COS cells, CHO cells and the like), and insect cells are mentioned.

When bacteria are used as hosts, it is preferable that while the recombinant vector can replicate autonomously in the bacteria, the recombinant vector comprise a promotor, a ribosome-binding sequence, an initiation codon, the nucleic acid encoding the improved protein of the present invention, and a transcription termination sequence. Examples of the colon bacilli include *Escherichia coli* DH5α. Examples of the hay bacilli include *Bacillus subtilis.* As long as the method for introducing the recombinant vector into bacteria is a method for introducing DNA into bacteria, the method is not particularly limited. For example, a method using calcium ions, electroporation, and the like are mentioned.

When the cyclic peptide is synthesized in cells, besides a peptide which is the substrate of cyclization reaction, a peptide relating to the folding of the peptide may be coexpressed. Examples of the peptide to be coexpressed include SlyA, TF (trigger factor), and peptidyl-prolyl isomerase (PPIase). For example, cyclophilin, FKBP, Pin1, and the like are mentioned, and, specifically, FKBP12 is mentioned.

In one aspect, the cyclic single-chain antibody of the present invention has an effect excellent in the suppression of aggregate formation as compared with the acyclic single-chain antibody. In one embodiment, the cyclic single-chain antibody has an effect excellent in the suppression of aggregate formation even in a solution obtained by redissolution after freeze-drying as compared with the acyclic single-chain antibody. In another aspect, the cyclic single-chain antibody has resistance also to damage by freeze-drying as compared with the acyclic single-chain antibody.

The present invention will be illustrated hereinafter by Examples, which are not intended to limit the present invention.

### Example 1

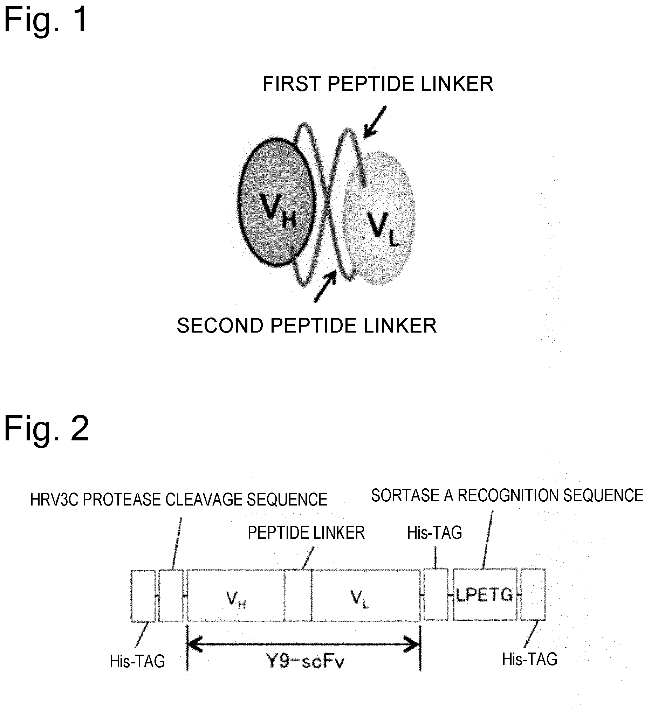

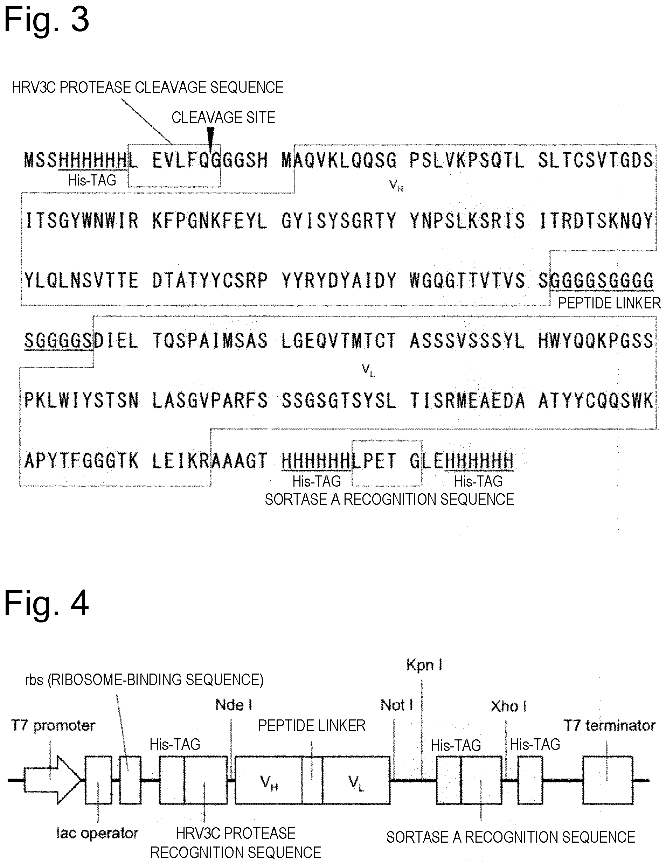

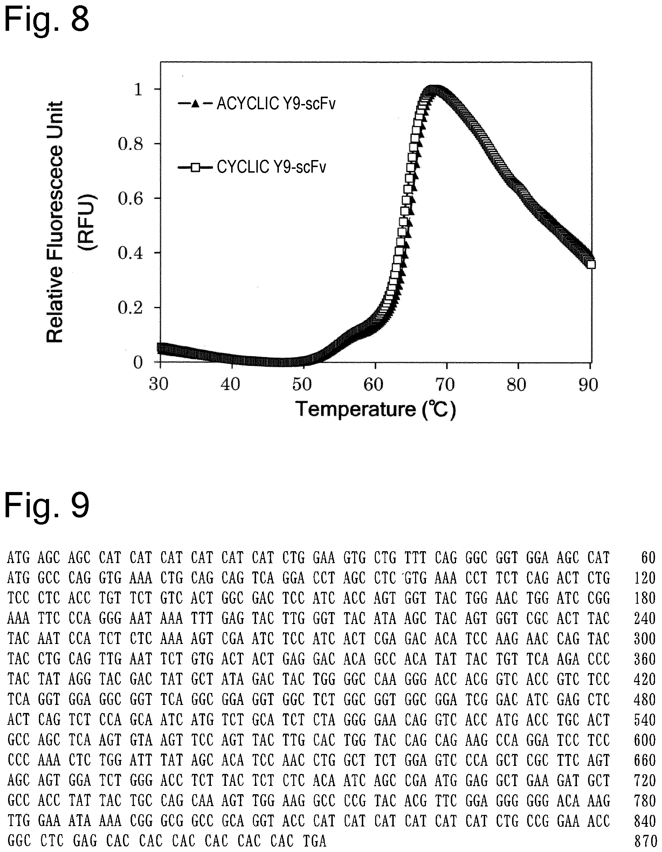

In the present Example, Y9-scFv, which specifically bound to GA-pyridine, was used. A polypeptide comprising His-tag, Y9-scFv, and an LPETG sequence from the N-terminus (Y9-scFv-LPETG, Fig. 2) was expressed by colon bacilli in the present Example. A plasmid in which a DNA fragment encoding a peptide of SEQ ID NO: 1 (Fig. 3) (Fig. 9) (pSAL-Y9-scFv, Fig. 4) was cloned into pET21-d (Merck KGaA) was provided.

Sortase A was prepared by a method described in a literature (J Biomol NMR. 43(3):145-150 (2009)).

A colon bacillus BL21 (DE3) was transformed by the plasmid pSAL-Y9-scFv. This colon bacillus is spread on LB agar medium containing ampicillin and incubated at 37°C all night, and the transformant was selected. One of the formed colonies was inoculated into LB medium containing 100 µg/ml ampicillin (20 ml) and precultured at 37°C for one night. This preculture solution was inoculated into LB medium containing 100 µg/ml ampicillin (300 ml) so that the turbidity at 600 nm was 0.1. When the turbidity at 600 nm reached 0.6, isopropyl-β-D(-)-thiogalactopyranoside was added at a final concentration of 1 mM, and culture was then performed at 37°C for 7 hours. The culture solution was centrifuged at 6000 rpm and 4°C for 10 minutes, and bacterial cells were collected. The bacterial cells were suspended in a sonication buffer (50 mM Tris-HCl (pH 8.0), 100 mM NaCl) and sonicated in ice. This solution was centrifuged at 6000 rpm and 4°C for 20 minutes, and the insoluble fraction was collected. This insoluble fraction was denatured with 6 M guanidine solution and then refolded to prepare Y9-scFv-LPETG maintaining three-dimensional structure.

Solubilization was performed by adding 15 mL of a solubilization buffer and mixing the mixture by inversion gently overnight. The sample after solubilization was centrifuged (12000 rpm, 4°C, 20 min), and the supernatant was collected. The collected supernatant was purified by Ni-NTA affinity chromatography (QIAGEN K.K.). Around 2 mL of Ni-NTA was packed in a column, and equilibrated with the solubilization buffer beforehand. The supernatant after centrifugation was loaded thereon, washing is performed by passing a washing buffer having 5 times the volume of Ni-NTA, and elution was finally performed with an elution buffer having 0.5 times the volume of Ni-NTA a total of 6 times. This was used for refolding.

Solubilization buffer: 6 M guanidine-HCl, 25 mM phosphate pH 7.4, 375 mM NaCl;
Washing buffer: 6 M guanidine-HCl, 25 mM phosphate pH 7.4, 375 mM NaCl, 10 mM imidazole;
Elution buffer: 6 M guanidine-HCl, 25 mM phosphate pH 7.4, 375 mM NaCl, 250 mM imidazole.

Refolding was performed by a method described in NPL 1. The specific procedure is shown below. The eluate from Ni-NTA containing the above-mentioned Y9-scFv-LPETG was diluted with the solubilization buffer so that the concentration of Y9-scFv-LPETG was 7.5 µmol/l. A dialysis tube having a molecular weight cut-off of around 14000 (SEKISUI MEDICAL CO., LTD.: 521768) was charged with this. Dialysis was performed against dialysis external liquid having 10 time the volume thereof. The dialysis external liquid was exchanged every 12 hours or every 24 hours. The following shows a procedure when the dialysis external liquid was exchanged every 12 hours. The volume of the dialysis external liquid is described as 1 L.
First day, first dialysis: dialysis external liquid: 3 M Gdn-HCl buffer: 1 L;
Second day, second dialysis: dialysis external liquid: 2/3 L + base buffer: 1/3 L;
   Third dialysis: 1 M Gdn-HCl buffer: 1 L + 375 µM oxidized glutathione; (NACALAI TESQUE, INC.);
Third day, fourth dialysis: dialysis external liquid: 500 mL + 1 M Gdn-HCl buffer: 500 mL + 375 µM oxidized glutathione;
   Fifth dialysis: dialysis external liquid: 500 mL + arginine base buffer 500 mL + 375 µM oxidized glutathione;
Fourth day, sixth dialysis: dialysis external liquid 500 mL + arginine base buffer 500 mL + 375 µM oxidized glutathione;
   Seventh dialysis: arginine base buffer: 1 L + 375 µM oxidized glutathione; Fifth day, eighth dialysis: base buffer: 1 L;

Then, the sample was collected. The compositions of the used buffers will be shown below.
3 M Gdn-HCl Buffer: 3 M guanidine-HCl, 50 mM Tris-HCl pH 7.5 at 4°C, 200 mM NaCl, 1 mM EDTA;
1 M Gdn-HCl buffer: 1 M guanidine-HCl, 50 mM Tris-HCl pH 7.5 at 4°C, 200 mM NaCl, 1 mM EDTA, 0.4 M L-Arginine;
Base buffer: 50 mM Tris-HCl pH 7.5 at 4°C, 200 mM NaCl, 1 mM EDTA
Arginine base buffer: 50 mM Tris-HCl pH 7.5 at 4°C, 200 mM NaCl, 1 mM EDTA, 0.4 M L-arginine;

Buffers were prepared at the time of use, and oxidized glutathione was added immediately before dialysis.

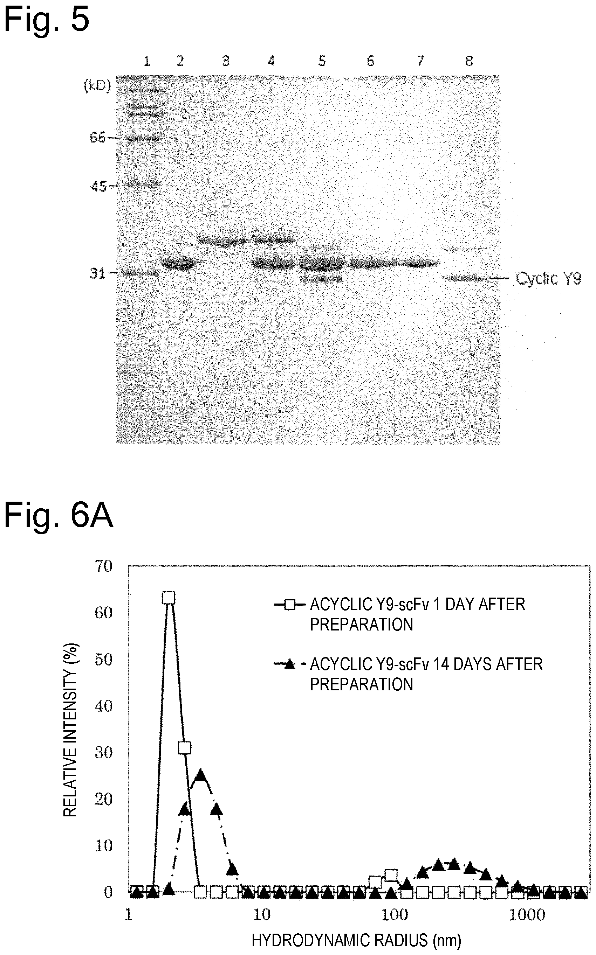

Then, HRV3C protease was added, and separation was performed by gel filtration chromatography to prepare Y9-scFv-LPETG which has glycine exposed at the N-terminus. Fifty mM HEPES, 150 mM NaCl, pH 7.4 was used for a running buffer of gel filtration chromatography. Fig. 5 shows the result of SDS-PAGE of cyclic Y9-scFv subjected to cyclization reaction using sortase A and purified. The lanes are as follows:
Lane 1: Molecular weight marker;
Lane 2: Sortase A;
Lane 3: Y9-scFv to which HRV3C protease was added;
Lane 4: Y9-scFv to which sortase A and HRV3C protease were added;
Lane 5: After cyclization reaction;
Lane 6: Fraction which passed without binding to Ni-NTA by Ni-NTA affinity chromatography;
Lane 7: Fraction at the time of washing; and
Lane 8: Eluted fraction.

Y9-scFv-LPETG and sortase A were mixed, cyclization reaction was performed at 25°C, and cyclic Y9-scFv was then separated by Ni-NTA affinity chromatography. Acyclic Y9-scFv was prepared by the method described in NPL 1 and used as a control.

Y9-scFv-LPETG (5 µM), sortase A (5 µM), and CaCl₂ (10 mM) were mixed into a buffer solution (5 mL; 50 mM HEPES, 150 mM NaCl, pH 7.4) in this composition, and the mixture was left to stand at 25°C for 1 hour to perform cyclization reaction. Then, cyclic Y9-scFv was separated by Ni-NTA affinity chromatography (Ni-NTA agarose resin; QIAGEN K.K.). The reaction liquid was applied to the column, equilibration was then performed using an equilibration solution (10 mL; 50 mM HEPES, 150 mM NaCl, pH 7.4), flow-through was obtained, washing was then performed with a washing solution (8 mL; 50 mM Tris-HCl (pH 8.0), 500 mM NaCl, 10 mM imidazole), and elution was then performed with an elution solution (5 mL; 50 mM Tris-HCl (pH 8.0), 500 mM NaCl, 250 mM imidazole) to obtain a substance of interest. When cyclization reaction was performed using 700 µg of Y9-scFv-LPETG, 500 µg of cyclic Y9-scFv was obtained.

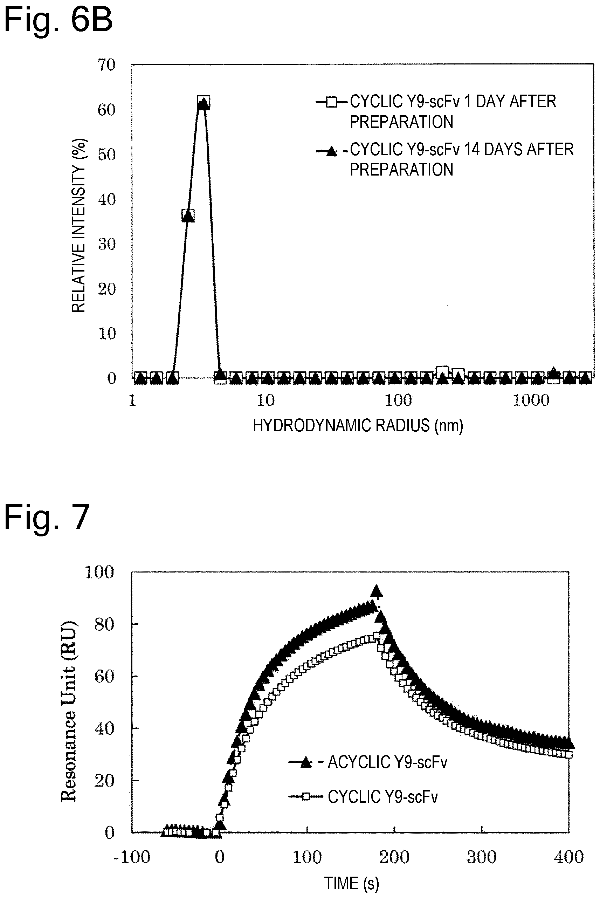

The stability (cohesiveness) was evaluated by dynamic light scattering measurement (DLS). A DynaPro NanoStar (Wyatt Technology Corporation) was used for measurement, and the measurement was performed at 25°C. Cyclic Y9-scFv and acyclic Y9-scFv used as a comparison object were concentrated by ultrafiltration. An Amicon Ultra 10 K 0.5 mL was used for concentration. The concentrated cyclic Y9-scFv and acyclic Y9-scFv were prepared using 50 mM HEPES, 150 mM NaCl, pH 7.4 so that the concentration was 3 mg/mL, and the preparations were left to stand at 4°C. One day and fourteen days after still standing, DLS measurement was performed, and the cohesivenesses were compared. Measurement was performed using supernatant fractions obtained after protein solutions left to stand at 4°C were centrifuged (15000 rpm, 30 min, 4°C), and impurities in the solutions were removed. Consequently, in cyclic Y9-scFv, 1 day after and even 14 days after still standing, only a peak at an inertial radius of around 3.2 nm is observed, and aggregates are hardly produced (Fig. 6B). Meanwhile, in acyclic Y9-scFv, 1 day after, molecular species having an inertial radius of around 2.6 nm is observed, a peak derived from aggregates having an inertial radius of around 110 nm is observed although the peak has low intensity, and a small amount of aggregates exist. Fourteen days after, the intensity of a peak derived from aggregates having an inertial radius of around 370 nm increased, and it became clear that aggregates were produced with time (Fig. 6A).

The antigen-binding activities of cyclic Y9-scFv and acyclic Y9-scFv were evaluated by surface plasmon resonance measurement. A Biacore T200 (GE Healthcare Corporation) was used for measurement. Measurement was performed by immobilizing a peptide containing GA-pyridine (Biotin-Gly-Ala-Gly-(GA-pyridine)-Gly-Ala-CONH₂) on the sensor chip SA (GE Healthcare Corporation). Cyclic Y9-scFv and acyclic Y9-scFv were prepared at 70 nM with an HBS-EP buffer under the condition of 25°C, and measurement was then performed. Consequently, the intensities and shapes of the sensorgrams were similar between cyclic Y9-scFv and acyclic Y9-scFv (Fig. 7), and it was confirmed that the antigen-binding activities were equivalent (Table 1).

[Table 1]

**Table 1**

| | Cyclic Y9-scFv | Acyclic Y9-scFv |
|---|---|---|
| *K*_{D}[×10⁻⁹(M)] | 16.1 ± 0.5 | 9.3 ± 0.3 |

The thermal stabilities of cyclic Y9-scFv and acyclic Y9-scFv were evaluated by differential scanning fluorescence measurement (DSF). Consequently, the shift of the thermal denaturation curve was not observed between cyclic Y9-scFv and acyclic Y9-scFv (Fig. 8), and it was confirmed that the thermal stabilities were also equivalent (Table 2).

[Table 2]

**Table 2**

| | Cyclic Y9-scFv | Acyclic Y9-scFv |
|---|---|---|
| *T*ₘ (°C) | 64.3 | 65.1 |

### Comparative Example 1

In Example 2, which is described below, Tras-scFv, which was an scFv derived from trastuzumab, which specifically bound to HER2, was used. Acyclic Tras-scFv, which is a comparative control of cyclic Tras-scFv, was prepared by the procedure described below.

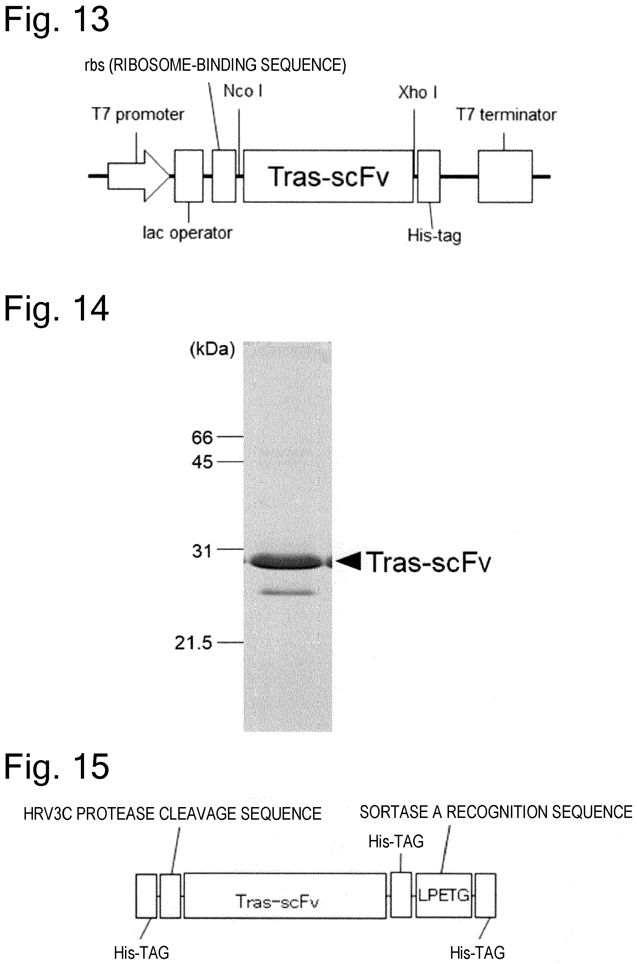

To express a polypeptide comprising Tras-scFv and His-tag sequence from the N-terminus (Tras-scFv, Fig. 11) by a colon bacillus, a plasmid in which a DNA fragment encoding the peptide of SEQ ID NO: 3 (Fig. 12)(SEQ ID NO: 4) was cloned into pET28 (Merck KGaA) (pET28-Tras-scFv, Fig. 13) was provided.

The colon bacillus BL21 (DE3) (Merck KGaA) was transformed by the plasmid pET28-Tras-scFv. This colon bacillus was spread on LB agar medium containing kanamycin and incubated at 37°C all night, and the transformant was selected. One of the formed colonies was inoculated into LB medium containing 50 µg/ml kanamycin (20 ml) and precultured at 37°C overnight. This preculture solution was inoculated into LB medium containing 50 µg/ml kanamycin (300 ml) so that the turbidity at 600 nm was 0.1. When the turbidity at 600 nm reached 0.6, isopropyl-β-D(-)-thiogalactopyranoside at a final concentration of 1 mM was added, and culture was then performed at 37°C for 7 hours. The culture solution was centrifuged at 6000 rpm and 4°C for 10 minutes, and the bacterial cells were collected. The bacterial cells were suspended in a sonication buffer (50 mM Tris-HCl (pH 8.0), 100 mM NaCl) and sonicated in ice. This solution was centrifuged at 6000 rpm and 4°C for 20 minutes, and the insoluble fraction was collected. This insoluble fraction was denatured and solubilized with a 6 M guanidine solution and then refolded to obtain Tras-scFv maintaining three-dimensional structure. Solubilization and refolding were performed by the technique described in Example 1.

Then, Tras-scFv was prepared by separation by gel filtration chromatography. A Superdex-75 (16/600) (GE Healthcare Corporation) was used for gel filtration chromatography, and 50 mM HEPES, 150 mM NaCl, pH 7.4 was used for a running buffer. Fig. 14 shows the result of SDS-PAGE of purified acyclic Tras-scFv.

### Example 2

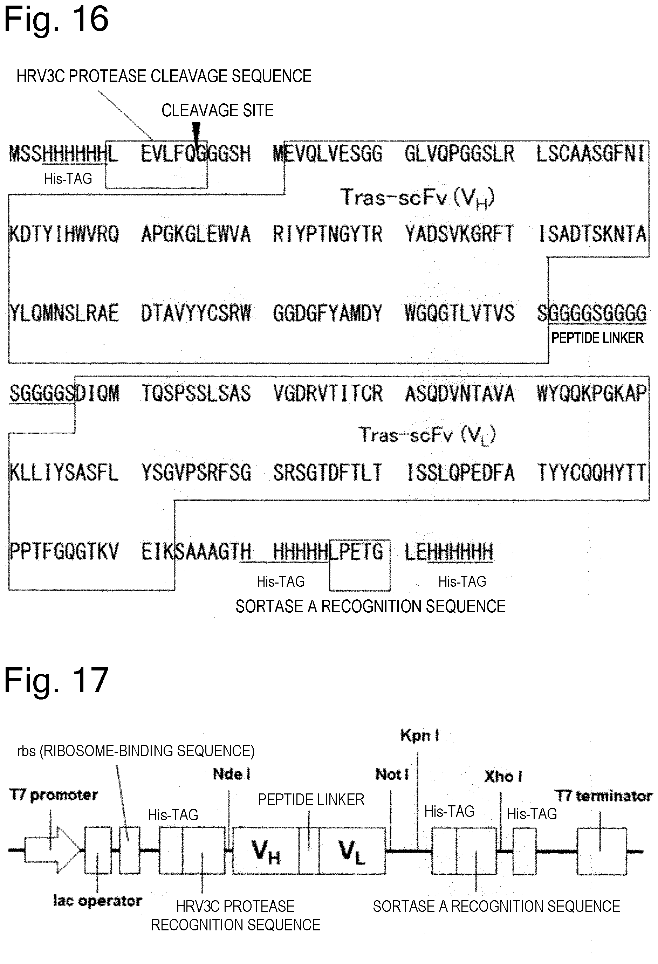

In the present Example, Tras-scFv, which was an scFv derived from trastuzumab, which specifically bound to HER2, was used. In the present Example, a polypeptide comprising His-tag, Tras-scFv, and LPETG sequence from the N-terminus (Tras-scFv-LPETG, Fig. 15) was expressed by a colon bacillus. A plasmid in which a DNA fragment encoding the peptide of SEQ ID NO: 5 (Fig. 16) (SEQ ID NO: 7) was cloned into pET21-d (pSAL-Tras-scFv, Fig. 17) was provided.

Sortase A was prepared by the method described in Example 1.

The colon bacillus BL21 (DE3) was transformed by the plasmid pSAL-Tras-scFv. This colon bacillus was spread on LB agar medium containing ampicillin and incubated at 37°C all night, and the transformant was selected. One of the formed colonies was inoculated into LB medium containing 100 µg/ml ampicillin (20 ml) and precultured at 37°C overnight. This preculture solution was inoculated into LB culture containing 100 µg/ml ampicillin (300 ml) so that the turbidity at 600 nm was 0.1. When the turbidity at 600 nm reached 0.6, isopropyl-β-D(-)-thiogalactopyranoside at a final concentration of 1 mM was added, and culture was then performed at 37°C for 7 hours. The culture solution was centrifuged at 6000 rpm and 4°C for 10 minutes, and the bacterial cells were collected. The bacterial cells were suspended in a sonication buffer (50 mM Tris-HCl (pH 8.0), 100 mM NaCl) and sonicated in ice. This solution was centrifuged at 6000 rpm and 4°C for 20 minutes, and the insoluble fraction was collected. This insoluble fragment was denatured and solubilized with a 6 M guanidine solution and then refolded to obtain Tras-scFv-LPETG maintaining three-dimensional structure. Solubilization and refolding were performed by the technique described in Example 1.

Then, HRV3C protease was added, and Tras-scFv-LPETG having glycine exposed at the N-terminus was prepared by separation by gel filtration chromatography. A Superdex-75 (16/600) (GE Healthcare Corporation) was used for gel filtration chromatography, and 50 mM HEPES, 150 mM NaCl, pH 7.4 was used for a running buffer. Tras-scFv-LPETG and sortase A were mixed, cyclization reacting was performed at 25°C, and cyclic Tras-scFv was then separated by Ni-NTA affinity chromatography.

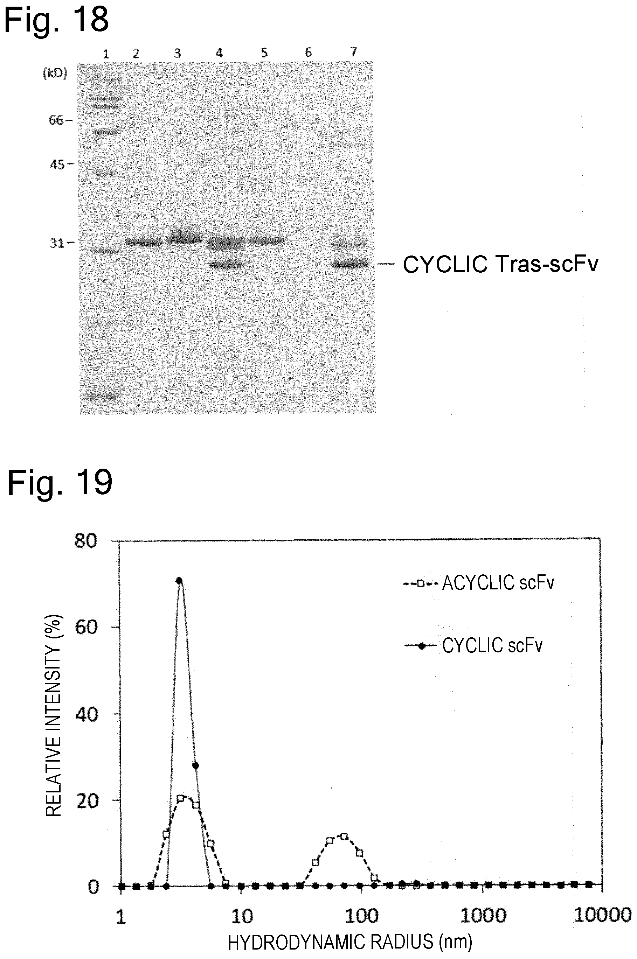

Fig. 18 shows the result of SDS-PAGE of cyclic Tras-scFv subjected to cyclization reaction using sortase A and purified. The lanes are as follows:
Lane 1: Molecular weight marker;
Lane 2: Sortase A;
Lane 3: Tras-scFv to which HRV3C protease was added;
Lane 4: After cyclization reaction;
Lane 5: Fraction which passed without binding to Ni-NTA by Ni-NTA affinity chromatography;
Lane 6: Fraction at the time of washing; and
Lane 7: Eluted fraction.

Tras-scFv-LPETG (5 µM), sortase A (5 µM), and CaCl₂ (10 mM) were mixed into a buffer solution (5 mL; 50 mM HEPES, 150 mM NaCl, pH 7.4) in this composition, and the mixture was left to stand at 25°C for 1 hour to perform cyclization reaction. Then, cyclic Tras-scFv was separated by Ni-NTA affinity chromatography (Ni-NTA agarose resin; Wako Pure Chemical Corporation). The reaction liquid was applied to the column, equilibration was then performed using an equilibration solution (10 mL; 50 mM HEPES, 150 mM NaCl, pH 7.4), flow-through was obtained, washing was then performed with a washing solution (8 mL; 50 mM Tris-HCl (pH 8.0), 500 mM NaCl, 10 mM imidazole), and elution was then performed with an elution solution (5 mL; 50 mM Tris-HCl (pH 8.0), 500 mM NaCl, 250 mM imidazole) to obtain a substance of interest. When cyclization reaction was performed using 700 µg of Tras-scFv-LPETG, 500 µg of cyclic Tras-scFv was obtained.

The stability (cohesiveness) was evaluated by dynamic light scattering measurement (DLS). A DynaPro NanoStar (Wyatt Technology Corporation) was used for measurement, and the measurement was performed at 25°C. Cyclic Tras-scFv and acyclic Tras-scFv used as a comparison object were concentrated by ultrafiltration. An Amicon Ultra 10 K 0.5 mL was used for concentration. The concentrated cyclic Tras-scFv and acyclic Tras-scFv were prepared using 50 mM HEPES, 150 mM NaCl, pH 7.4 so that the concentrations were 3 mg/mL, and the preparations were left to stand at 4°C. One day after still standing, DLS measurement was performed, and the cohesivenesses were compared. Measurement was performed using supernatant fractions obtained after protein solutions left to stand at 4°C were centrifuged (15000 rpm, 30 min, 4°C), and impurities in the solutions were removed. Consequently, even 1 day after still standing, in cyclic Tras-scFv, only a peak at an inertial radius of around 3.2 nm is observed, and aggregates are hardly produced (Fig. 19). Meanwhile, in acyclic Tras-scFv, 1 day after still standing, molecular species having an inertial radius of around 2.6 nm are observed, a peak derived from aggregates having an inertial radius of around 100 nm is observed although the peak has low intensity, and aggregates exist.

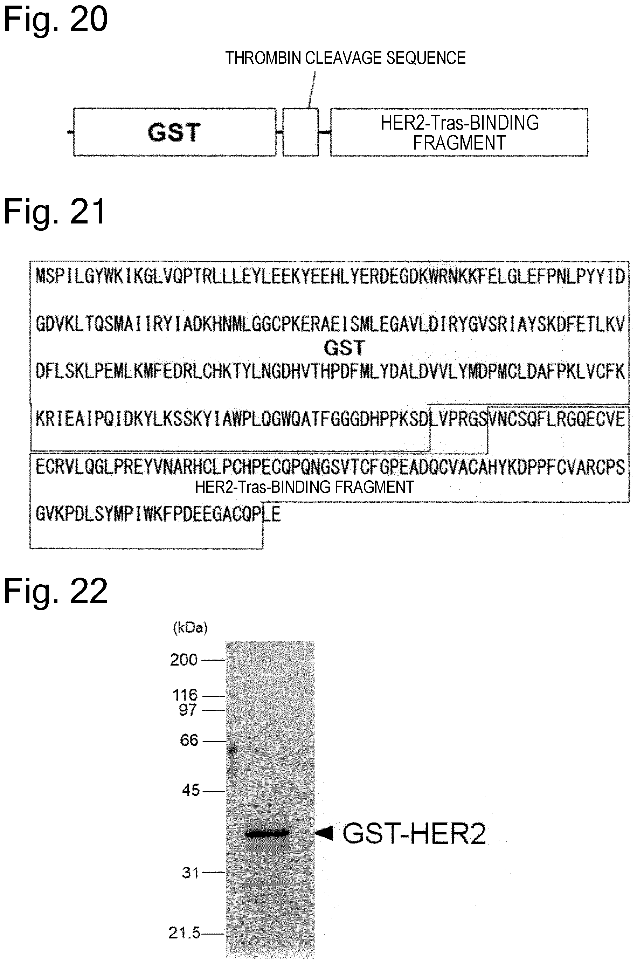

To evaluate the antigen-binding activities of cyclic Tras-scFv and acyclic Tras-scFv by surface plasmon resonance measurement, a fusion protein of a peptide fragment (HER2-Tras-binding fragment) containing the trastuzumab-binding region of HER2 and glutathione S-transferase (GST) (GST-HER2) was prepared. To express the polypeptide comprising GST and HER2-Tras-binding fragment sequence from the N-terminus (GST-HER2, Fig. 20) by a colon bacillus, a plasmid in which a DNA fragment encoding a peptide of SEQ ID NO: 8 (Fig. 21) (SEQ ID NO: 9) was cloned into pGEX4T-3 (pGEX4T-HER2) was provided. The colon bacillus SHuffle-T7 (NEW ENGLAND BioLabs Inc.) was transformed by the plasmid pGEX4T-HER2. This colon bacillus was spread on LB agar medium containing 100 µg/ml ampicillin and 50 µg/mL streptomycin and incubated at 37°C all night, and the transformant was selected. One of the formed colonies was inoculated into LB medium containing 100 µg/ml ampicillin and 50 µg/mL streptomycin (20 ml) and precultured at 37°C overnight. This preculture solution was subcultured in TB medium containing 100 µg/ml ampicillin and 50 µg/mL streptomycin (300 ml). When the turbidity at 600 nm reached 2.5 to 3, isopropyl-β-D(-)-thiogalactopyranoside at a final concentration of 1 mM was added, rapid cooling to 15°C was then performed in ice bath, and culture was performed at 15°C for 72 hours. The culture solution was centrifuged at 6000 rpm and 4°C for 10 minutes, and the bacterial cells were collected. The bacterial cells were suspended in a sonication buffer (50 mM Tris-HCl (pH 8.0), 100 mM NaCl) and sonicated in ice. This solution was centrifuged at 6000 rpm and 4°C for 20 minutes, and the supernatant was collected. GST-HER2 was separated from the collected supernatant by affinity chromatography using a Glutathione Sepharose 4B (GS-4B) (GE Healthcare Corporation). The collected supernatant was applied to a GS-4B affinity chromatography column, washing was then performed with a washing solution (8 mL; 50 mM Tris-HCl (pH 8.0), 150 mM NaCl), and elution was then performed with an elution solution (5 mL; 50 mM Tris-HCl (pH 8.0), 150 mM NaCl, 30 mM reduced glutathione) to obtain a substance of interest. The product obtained by GS-4B affinity chromatography was further purified by gel filtration chromatography. A Superdex-200 (16/600) (GE Healthcare Corporation) was used for gel filtration chromatography, and 50 mM HEPES, 150 mM NaCl, pH 7.4 was used for a running buffer. Fig. 22 shows the result of SDS-PAGE of purified GST-HER2.

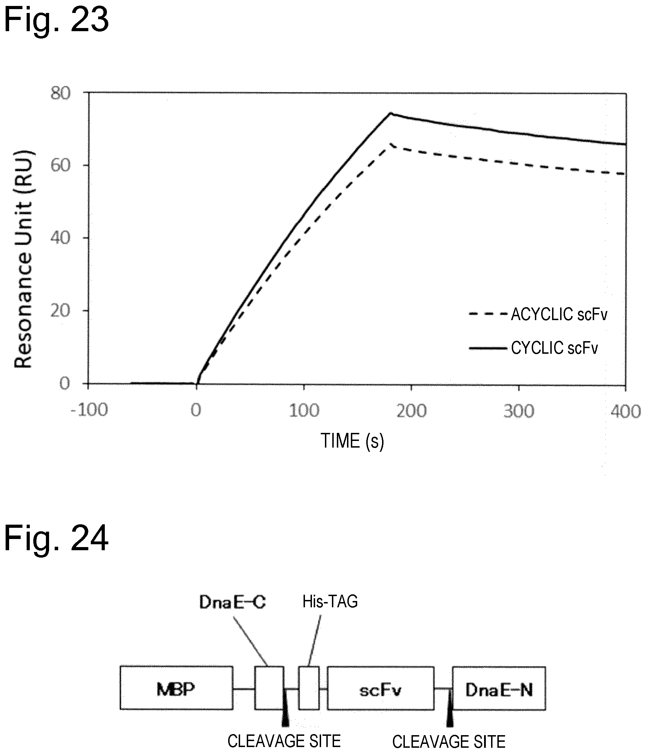

The antigen-binding activities of cyclic Tras-scFv and acyclic Tras-scFv were evaluated by surface plasmon resonance measurement. A Biacore T200 (GE Healthcare Corporation) was used for measurement. Measurement was performed by immobilizing a fusion protein of a peptide fragment containing the trastuzumab-binding region of HER2, and glutathione S-transferase (GST) (GST-HER2) on a sensor chip. A Series S Sensor Chip CM5 (GE Healthcare Corporation) was used for the sensor chip, and a GST Capture Kit (GE Healthcare Corporation) was used for immobilizing GST-HER2. Cyclic Tras-scFv and acyclic Tras-scFv were adjusted to 70 nM with an HBS-EP buffer under the condition of 25°C, and measurement was then performed. Consequently, the intensities and shapes of the sensorgrams were similar between cyclic Tras-scFv and acyclic Tras-scFv (Fig. 23), and it was confirmed that the antigen-binding activities were equivalent (Table 3).

[Table 3]

**Table 3**

| | Cyclic Tras-scFv | Acyclic Tras-scFv |
|---|---|---|
| *K*_{D}[×10⁻⁹(M)] | 1.28±0.02 | 1.15±0.03 |

### Example 3

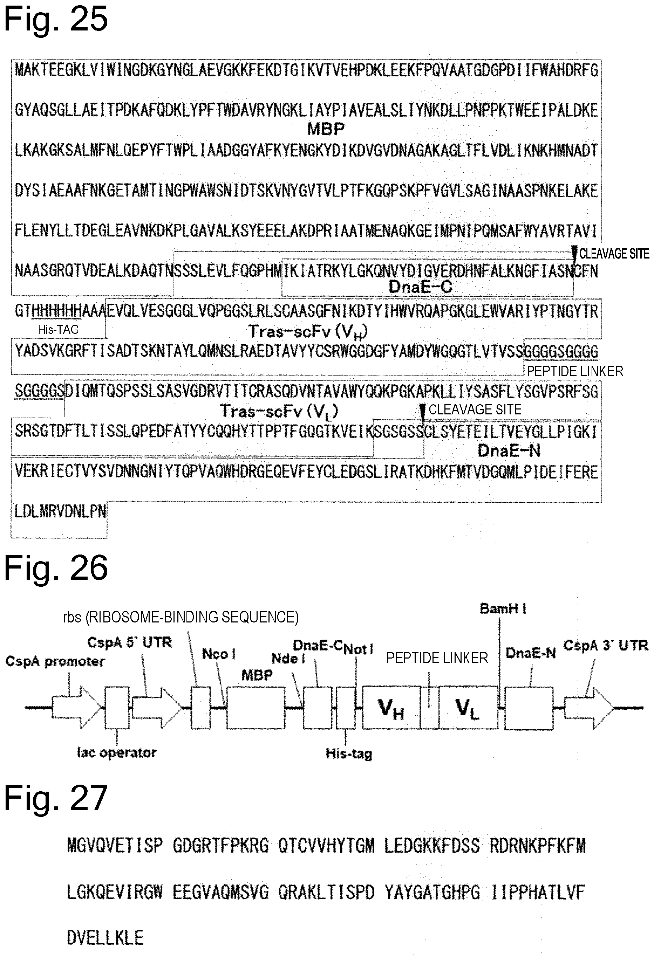

In the present Example, Tras-scFv, which was an scFv derived from trastuzumab, which specifically bound to HER2, was used. Cyclization reaction was performed in bacterial cells of a colon bacillus using intein reaction. In the present Example, a polypeptide comprising maltose-binding protein (MBP), DnaE-C, His-tag, Tras-scFv, and DnaE-N from the N-terminus (Tras-scFv-Intein, Fig. 24) was expressed by the colon bacillus. A plasmid in which a DNA fragment encoding the peptide of SEQ ID NO: 10 (Fig. 25) (SEQ ID NO: 12) was cloned into the plasmid pCold-NcoI, which was described in NON PATENT LITERATURE (Kobashigawa Y et al., Genes Cells, 20, 860-870 (2015)) (pNMK-Tras-scFv, Fig. 26) was provided.

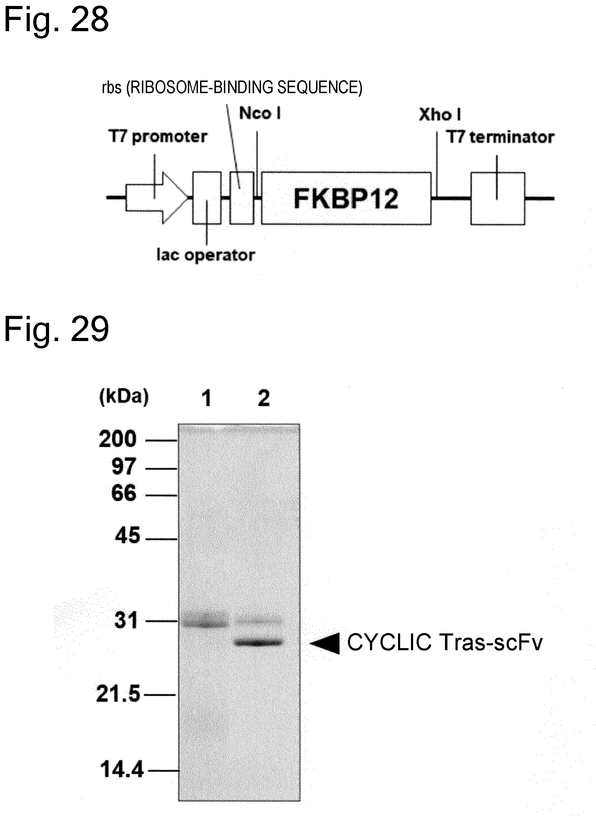

A plasmid in which the DNA fragment encoding FKBP12, which was set forth in SEQ ID NO: 13 and Fig. 27, was cloned into pET21-d (pET21-FKBP12: Fig. 28) was provided.

The colon bacillus strain SHuffle-T7 (NEW ENGLAND BioLabs Inc.) was transformed by pNMK-Tras-scFv, pET21-FKBP12, and pG-KJE8 (Takara Bio Inc.). This colon bacillus was spread on LB agar medium containing 100 µg/ml ampicillin, 50 µg/ml kanamycin, 30 µg/ml chloramphenicol, and 50 µg/ml streptomycin and incubated at 37°C all night, and the transformant was selected. One of the formed colonies was inoculated into TB medium containing 100 µg/ml ampicillin, 50 µg/ml kanamycin, 30 µg/ml chloramphenicol, and 50 µg/ml streptomycin (20 ml) and precultured at 37°C overnight. This preculture solution was subcultured in TB medium containing 100 µg/ml ampicillin, 50 µg/ml kanamycin, 30 µg/ml chloramphenicol, 50 µg/ml streptomycin, and 5 ng/ml tetracycline (300 ml). When the turbidity at 600 nm reached 2.5 to 3, isopropyl-β-D(-)-thiogalactopyranoside at a final concentration of 1 mM was added, rapid cooling to 15°C was then performed in ice bath, and culture was further performed for 72 hours. The culture solution was centrifuged at 6000 rpm and 4°C for 10 minutes, and the bacterial cells were collected. The bacterial cells were suspended in a sonication buffer (50 mM Tris-HCl (pH 8.0), 100 mM NaCl) and sonicated in ice. This solution was centrifuged at 6000 rpm and 4°C for 20 minutes, and the supernatant was collected. Cyclic Tras-scFv was separated from the collected supernatant by Ni-NTA affinity chromatography (Ni-NTA agarose resin; Wako Pure Chemical Corporation). The collected supernatant was applied to an Ni-NTA affinity chromatography column, washing was then performed with a washing solution (8 mL; 50 mM Tris-HCl (pH 8.0), 500 mM NaCl, 10 mM imidazole), and elution was then performed with an elution solution (5 mL; 50 mM Tris-HCl (pH 8.0), 500 mM NaCl, 250 mM imidazole) to obtain a substance of interest. The product obtained by Ni-NTA was further purified by gel filtration chromatography. A Superdex-75 (16/600) (GE Healthcare Corporation) was used for gel filtration chromatography, and 50 mM HEPES, 150 mM NaCl, pH 7.4 was used for a running buffer. Fig. 29 shows the result of SDS-PAGE of cyclic Tras-scFv subjected to cyclization using an intein reaction and purified.

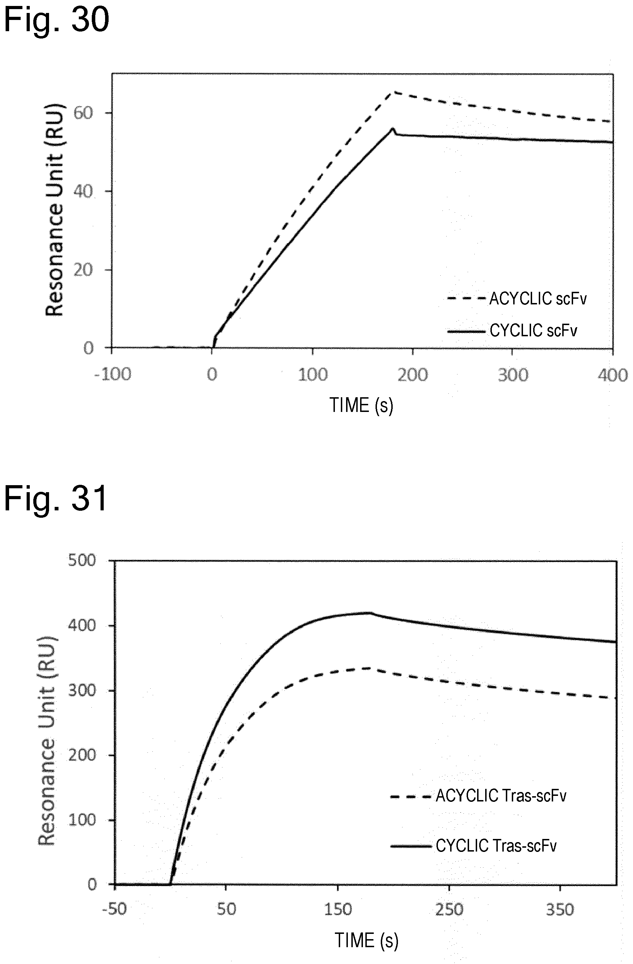

The antigen-binding activities of cyclic Tras-scFv and acyclic Tras-scFv were evaluated by surface plasmon resonance measurement. A Biacore T200 (GE Healthcare Corporation) was used for measurement. Measurement was performed by immobilizing a fusion protein of a peptide fragment containing the trastuzumab-binding region of HER2, and glutathione S-transferase (GST) (GST-HER2) on a sensor chip. A Series S Sensor Chip CM5 (GE Healthcare Corporation) was used for the sensor chip, and a GST Capture Kit (GE Healthcare Corporation) was used for immobilizing GST-HER2. Cyclic Tras-scFv and acyclic Tras-scFv were prepared with an HBS-EP buffer at 70 nM under the condition of 25°C, and measurement was then performed. Consequently, the intensities and shapes of the sensorgrams were similar between cyclic Tras-scFv and acyclic Tras-scFv (Fig. 30), and it was confirmed that the antigen-binding activities were equivalent (Table 4).

[Table 4]

**Table 4**

| | Cyclic Tras-scFv | Acyclic Tras-scFv |
|---|---|---|
| *K*_{D}[×10⁻⁹(M)] | 0.59 ± 0.01 | 1.28 ± 0.02 |

### Example 4

In the present Example, freeze-drying was preformed using cyclic Tras-scFv and acyclic Tras-scFv prepared in Example 2, and the amounts of aggregates formed at the time of redissolution in water thereafter were compared. The amount of aggregates can be observed by measuring the absorbance at 360 nm. As the amount of aggregates becomes larger, the absorbance at 360 nm becomes higher.

Cyclic or acyclic Tras-scFv dissolved in a buffer solution (50 mM HEPES, pH 7.4, 150 mM NaCl) was concentrated to 1.3 mg/mL by ultrafiltration. Five hundred µL of this solution was prepared. This solution was dialyzed against 1 L of a dialysis external liquid (10 mM sodium citrate, 90 mM sucrose, pH 6.2). The liquid in the dialysis membrane was collected, the concentration was then quantified again, and 400 µL of the 1.3 mg/mL scFv solution was prepared at a concentration of 1.3 mg/mL in a 1.5-mL microtube. This solution was frozen in liquid nitrogen and freeze-dried. It was confirmed that the solvent sublimated completely, and 85 µL of ultrapure water was added, and still standing was performed at 4°C for around 1 hour, and redissolution was performed by occasional gentle mixing with a pipette. The concentration after redissolution was around 6 mg/mL. The absorbance at 360 nm was measured as to this solution. Consequently, it was found that the absorbance at 360 nm of cyclic Tras-scFv is low, and the amount of aggregates was small (Table 5) as compared with acyclic Tras-scFv. The antigen-binding activities of cyclic Tras-scFv before freeze-drying and redissolved after freeze-drying were measured by surface plasmon resonance measurement, and it was confirmed that the antigen affinities were equivalent (Table 6). Cyclic Tras-scFv and acyclic Tras-scFv after freeze-drying were prepared at a concentration of 40 nM, and the maximum RU values in surface plasmon resonance measurement were compared. The maximum RU value is an index of the residual activity of Tras-scFv at the time of redissolution after freeze-drying, and it is shown that as the maximum RU value becomes larger, the residual activity becomes higher. Consequently, it was found that cyclic Tras-scFv had a large maximum RU value, and cyclic Tras-scFv had higher residual activity after freeze-drying (Table 7) as compared with acyclic Tras-scFv.

[Table 5]

**Table 5**

| | Cyclic Tras-scFv | Acyclic Tras-scFv |
|---|---|---|
| Absorbance at 360 nm | 0.033 ± 0.002 | 0.105 ± 0.004 |

[Table 6]

**Table 6**

| | **Cyclic Tras-scFv before freeze-drying** | **Cyclic Tras-scFv redissolved after freeze-drying** |
|---|---|---|
| *K*_{D}[×10⁻⁹(M)] | 1.3 | 1.8 |

[Table 7]

**Table 7**

| | **Cyclic Tras-scFv** | **Acyclic Tras-scFv** |
|---|---|---|
| **Maximum RU value** | **421** | **336** |

## Claims

1. A single-chain antibody (scFv) comprising a heavy chain variable region (VH) and a light chain variable region (VL) linked by a first peptide linker, wherein an N-terminus and a C-terminus of the single-chain antibody are linked by a second peptide linker to form a cyclic single-chain antibody.

2. The single-chain antibody according to claim 1, wherein the cyclic single-chain antibody has a cyclic structure formed by only peptide bonds.

3. The single-chain antibody according to claim 1 or 2, wherein the VH and the VL are associated in a molecule to form an antigen-binding site.

4. The single-chain antibody according to any one of claims 1 to 3, wherein the cyclic single-chain antibody has one antigen-binding site in a molecule.

5. The single-chain antibody according to any one of claims 1 to 4, wherein the first peptide linker consists of 15 to 27 amino acids.

6. The single-chain antibody according to any one of claims 1 to 5, wherein the second peptide linker consists of 15 to 28 amino acids.

7. The single-chain antibody according to any one of claims 1 to 6, wherein aggregate formation is suppressed as compared with an acyclic single-chain antibody having the same VH and the same VL.

8. The single-chain antibody according to any one of claims 1 to 7, wherein the second peptide linker is formed by transpeptidase.

9. The single-chain antibody according to claim 8, wherein the transpeptidase is sortase.

10. The single-chain antibody according to any one of claims 1 to 9, wherein the second peptide linker comprises an amino acid sequence: LPXTG wherein X represents any amino acid residue.

11. The single-chain antibody according to any one of claims 1 to 7, wherein the second peptide linker is formed by trans-splicing reaction by a split intein.

12. A method for producing the cyclic single-chain antibody according to any one of claims 1 to 7, comprising the steps of:
1) preparing an acyclic peptide which is a single-chain antibody (scFv) comprising a heavy chain variable region (VH) and a light chain variable region (VL) linked by a first peptide linker, and has transpeptidase recognition sequences at the N-terminus and the C-terminus; and
2) forming a second peptide linker from the transpeptidase recognition sequences of the N-terminus and the C-terminus of the single-chain antibody using transpeptidase and cyclizing the single-chain antibody.

13. The production method according to claim 8, wherein the transpeptidase is sortase.

14. The production method according to claim 12 or 13, wherein the transpeptidase recognition sequence of the N-terminus of the acyclic peptide comprises LPXTG wherein X represents any amino acid residue.

15. The production method according to any one of claims 12 to 14, wherein the transpeptidase recognition sequence of the C-terminus of the acyclic peptide comprises GG.

16. A method for producing the cyclic single-chain antibody according to any one of claims 1 to 7, comprising the steps of:
1) preparing an acyclic peptide which is a single-chain antibody (scFv) comprising a heavy chain variable region (VH) and a light chain variable region (VL) linked by the first peptide linker, and has a C-terminal split intein fragment (Int-C) at the N-terminus and an N-terminal split intein fragment (Int-N) at the C-terminus; and
2) forming a second peptide linker by trans-splicing reaction by the split intein and cyclizing the single-chain antibody.

17. The production method according to claim 16, wherein the C-terminal split intein fragment (Int-C) used is DnaE-Int-C, and the N-terminal split intein fragment (Int-N) used is DnaE-Int-N.

18. A nucleic acid comprising a nucleotide sequence encoding an amino acid sequence of the acyclic peptide according to step 1 of claim 16.

19. A recombinant vector comprising the nucleic acid according to claim 18.

20. A transformant comprising the recombinant vector according to claim 19 introduced thereinto.
